(19) Europäisches Patentamt
European Patent Office
Office européen des brevets

(11) Veröffentlichungsnummer: **0 133 673**
**B1**

(12) # EUROPÄISCHE PATENTSCHRIFT

(45) Veröffentlichungstag der Patentschrift: **24.06.87**

(51) Int. Cl.⁴: **A 61 K 31/35,** C 07 D 311/94 // A61K35/78

(21) Anmeldenummer: **84108898.2**

(22) Anmeldetag: **27.07.84**

(54) **Neue valepotriathydrinhaltige Arzneimittel.**

(30) Priorität: **02.08.83 DE 3327811**

(43) Veröffentlichungstag der Anmeldung: **06.03.85 Patentblatt 85/10**

(45) Bekanntmachung des Hinweises auf die Patenterteilung: **24.06.87 Patentblatt 87/26**

(84) Benannte Vertragsstaaten: **AT BE CH DE FR GB IT LI NL**

(56) Entgegenhaltungen:
**EP - A - 0 011 718**
**DE - A - 1 961 433**
**DE - A - 2 654 709**
**DE - A - 3 026 579**
**DE - B - 2 230 626**
**DE - C - 1 191 515**

**PATENT ABSTRACTS OF JAPAN, unexamined applications, Sektion C, Band 5, Nr. 89, 10. Juni 1981 THE PATENT OFFICE JAPANESE GOVERNMENT Seite 92 C 58, Kokai-Nr. 56-34 627**
**PATENT ABSTRACTS OF JAPAN, unexamined applications, Sektion C, Band 5, Nr. 160, 15. Oktober 1981 THE PATENT OFFICE JAPANESE GOVERNMENT Seite 40 C 75, Kokai Nr. 56-90 011**
**TETRAHEDRON LETTERS, Jahrgang 1976, Nr. 15,**

(73) Patentinhaber: **Kali-Chemie Pharma GmbH, Hans-Böckler-Allee 20 Postfach 220, D-3000 Hannover 1 (DE)**

(72) Erfinder: **Thies, Peter Willibrord, Dr.Phil, Ihmeplatz 6, D-3000 Hannover 91 (DE)**
Erfinder: **David, Samuel, Dr., Dipl.-Chem., Tiergartenstrasse 103c, D-3000 Hannover 73 (DE)**
Erfinder: **Hell, Insa, Dr., Wiesenstrasse 13, D-3000 Hannover 1 (DE)**
Erfinder: **Wolf, Klaus-Ullrich, Dr., Imkersweg 6, D-3165 Hänigsen (DE)**

(74) Vertreter: **Lauer, Dieter, Dr., c/o Kali-Chemie AG Postfach 220 Hans-Böckler-Allee 20, D-3000 Hannover 1 (DE)**

(56) Entgegenhaltungen: *(Fortsetzung)*
**Oxford, New York, Paris, Frankfurt, PERGAMON PRESS J. HÖLZL et al. "Zur Struktur von drei gemeinen Valtrathydrinen aus Valeriana Tiliacfolia", S.1171-1174**
**COMPTES RENDUS DE L'ACADEMIE BULGARE DES SCIENCES, Band 28, Nr. 5, 1975, ACAD. BULG. SCI, Sofija S.S. POPOV et al. "Biogenetic Relationship Between Some Valepotriates" Seiten 651-653**
**TETRAHEDRON, Band 29, Nr. 15, 1973, Oxford, London, New York, Paris, PERGAMON PRESS P. W. THIES "Über die Wirkstoffe des Baldrians-X" Seiten 3213-3226**
**TETRAHEDRON, Band 24, 1968, Oxford, London, New York, Paris, PERGAMON PRESS P.W. THIES "Die Konstitution der Valepotriate" Seiten 313-347**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft die Verwendung von Valepotriathydrinen zur Herstellung von pharmazeutischen Zubereitungen zur Prophylaxe und Behandlung von peptischen Ulcera und/oder zur Behandlung von krampfartigen Motilitätsstörungen im gastrointestinalen Trakt in grösseren Säugetieren und Menschen und die Herstellung von für diese Behandlungen geeigneten Arzneimitteln, welche als Wirkstoffe Valepotriathydrine enthalten sowie neue Valepotriathydrine mit peptische Ulcusbildung hemmenden und motilitätsnormalisierenden Eigenschaften.

Die erfindungsgemäss verwendeten Valepotriathydrine stellen Polyacyloxycyclopenta(c)pyran-Verbindungen der allgemeinen Formel I dar

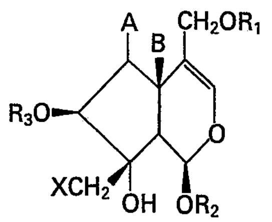

I

worin A für Wasserstoff und B für Wasserstoff oder Hydroxy stehen oder A und B gemeinsam eine Bindung bilden, von den Resten $R_1$ bis $R_3$ einer für Isovaleroyl, ein weiterer für Acetyl und der dritte für den Acylrest einer der folgenden Säuren steht: Isovaleriansäure, β-Methylvaleriansäure, α-Isovaleroyloxyisovaleriansäure, α-Acetoxyisovaleriansäure, β-Acetoxyisovaleriansäure, β-Acetoxy-β-methylvaleriansäure oder β-Hydroxyisovaleriansäure, und X für Halogen, Cyano, Rhodano, Azido oder einen Acyloxyrest $R_4COO$ steht, worin $R_4$ eine gegebenenfalls durch Hydroxy substituierte Alkylgruppe mit 1 bis 20 Kohlenstoffatomen, eine Alkenylgruppe mit 3–20 Kohlenstoffatomen oder eine Phenyl-, Phenylalkyl- oder Phenylalkenylgruppe mit bis zu 3 Kohlenstoffatomen in der Alkylen- oder Alkenylenkette, welche gegebenenfalls im Phenylring durch Halogen, Trifluormethyl, Hydroxy, niederes Alkyl oder niederes Alkoxy substituiert sein kann, bedeutet.

Valepotriate sind Verbindungen der allgemeinen Formel II

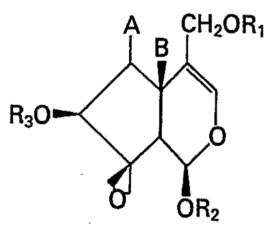

II

worin A, B und $R_1$ bis $R_3$ die obige für Formel I angegebene Bedeutung besitzen. Valepotriate sind bekannt als pharmakologisch aktive Inhaltsstoffe des Baldrians, bzw. von Pflanzen aus der Familie der Valerianaceen [siehe z.B. Thies in Tetrahedron 24, 313 bis 317 (1968) und Thies et al in Planta medica 41, 15 bis 20 (1981)]. Zu den bekanntesten Valepotriaten zählen Valtratum (A+B = eine Bindung, $R_1$ = Acetyl, $R_2$ = Isovaleroyl, $R_3$ = Isovaleroyl), Isovaltratum (A+B = Bindung, $R_1$ = Isovaleroyl, $R_2$ = Isovaleroyl, $R_e$ = Acetyl), Acevaltratum (A+B = Bindung, $R_1$ = Acetyl, $R_2$ = Isovaleroyl, $R_3$ = β-Acetoxyisovaleroyl) und Didrovaltratum (A+B = je Wasserstoff, $R_1$ = Isovaleroyl, $R_2$ = Isovaleroyl, $R_3$ = Acetyl). Vielfach liegen in aus Pflanzen gewonnenen Valepotriatgemischen gemeinsam mit diesen Valepotriaten auch analoge Homoverbindungen, worin ein Isovaleroylrest durch β-Methylvaleroyl ersetzt ist, vor. Die Valepotriate sind bekannt für ZNS-wirksame Eigenschaften, insbesondere beruhigende und psychisch ausgleichende und auch spasmolytische Eigenschaften. Diese Wirkungen des Baldrians und seiner Inhaltsstoffe werden schon seit langem pharmazeutisch angewandt.

Im Rahmen von Arbeiten zur Aufklärung der Konstitution bzw. zur Herstellung von chemischen Derivaten von Valepotriaten sind einige Halogen- und Rhodano-Hydrine der vier vorstehend genannten Valepotriate als Zwischenprodukte hergestellt und beschrieben worden. So sind z.B. die Jodhydrine, Rhodanohydrine und Bromhydrine des Valtratum, Acevaltratum und des Didrovaltratum (siehe Tetrahedron 24, 313–317 und die DE-OS 1961433 und 3026579), das Chlorhydrin des Valtratum [siehe C.R. acad. Bulg. 28, 651 (1979)] und das Jodhydrin und das Rhodanohydrin des Isovaltratum [siehe Tetrahedron 19, 2213–3226 (1973)] als Zwischenprodukte bekannt geworden. Ebenfalls als Zwischenprodukt bekannt geworden ist das Acetoxyhydrin des Didrovaltratum. Ferner sind einige Hydrine der bekanntesten Valepotriate, in welchen X den Säurerest einer natürlich in Baldriangewächsen vorkommenden Säure darstellt, insbesondere die Isovaleroyloxyhydrine des Valtratum, des Acevaltratum, des Isovaltratum und des Didrovaltratum sowie die Acetoxyhydrine des Valtratum und des Isovaltratum [siehe Tetrahedron Letters (1976) 1171] beschrieben worden. Für die als Zwischenprodukte hergestellten und sonst namentlich beschriebenen Valepotriathydrine ist bisher keine pharmakologische Wirksamkeit bekannt geworden.

In der DE-OS 3112732 wird ein Verfahren zur Gewinnung eines epoxidfreien, sedierende Wirkstoffe enthaltenden Extraktes aus Baldrian beschrieben, welcher u.a. auch Valepotriathydrine enthalten soll. Es werden aber keine einzelnen Valepotriathydrine oder deren Wirkung genauer beschrieben.

Der Erfindung liegt die Aufgabe zugrunde, neue Arzneimittel zur Behandlung und Prophylaxe von Erkrankungen des gastrointestinalen Traktes zu entwickeln.

Ferner liegt der Erfindung die Aufgabe zugrunde, Valepotriathydrine mit wertvollen pharmakologischen Eigenschaften herzustellen.

Es wurde nun überraschend gefunden, dass die Valepotriathydrine der Formel I wertvolle pharmakologische Eigenschaften, insbesondere eine günstige pharmakologische Wirkung im gastrointestinalen Trakt besitzen. Sie zeichnen sich durch eine Schutzwirkung auf die gastrointestinale Schleimhaut und durch eine Hemmwirkung auf peptische Ulcusbildung und/oder durch gute Wirksamkeit gegen krampfartige Motilitätsstörungen bei einer guten Verträglichkeit und nur geringer Toxizität aus.

Aufgrund ihrer Wirkungen im gastrointestinalen Trakt von grösseren Säugetieren und Menschen sind die Valepotriathydrine der Formel I zur Verwendung als Arzneimittel in der Gastroenterologie geeignet.

Als erfindungsgemässe Wirkstoffe können einzelne Valepotriathydrine oder Valepotriathydringemische verwendet werden, z.B. Hydringemische, welche aus aus Extrakten von Wurzeln und Rhizomen verschiedener Valerianaceen- und Kenthrantusarten gewonnenen Valepotriatgemischen hergestellt werden.

So werden zweckmässigerweise Valepotriathydrine der Formel I verwandt, worin $R_1$ Isovaleroyl oder α-Isovaleroyloxyisovaleroyl und $R_3$ Acetyl oder $R_1$ Acetyl und $R_3$ Isovaleroyl oder auch β-Acetoxyisovaleroyl bedeuten und $R_2$ Isovaleroyl oder β-Methylvaleroyl bedeutet. Unter den Valepotriathydrinen mit Dienstruktur (A+B = Bindung) kommen zweckmässigerweise die Hydrine des Valtratum, Isovaltratum, Homovaltratum, Homoisovaltratum, Acevaltratum und Homoacevaltratum oder deren Gemische in Frage. So eignen sich insbesondere deren Chlorhydrine, Acetoxyhydrine und Isovaleroyloxyhydrine.

Unter den Valepotriathydrinen mit Monoenstruktur (A+B je Wasserstoff) kommen zweckmässigerweise die Hydrine des Didrovaltratum, des Homodidrovaltratum oder von deren Gemischen in Frage. So eignen sich z.B. insbesondere Didrovaltrathalogenhydrine, vorzugsweise Didrovaltratchlorhydrin, Didrovaltratalkanoyloxyhydrine, vorzugsweise Didrovaltratisovaleroyloxyhydrin oder Didrovaltratacetoxyhydrin, aromatische Didrovaltratacyloxyhydrine, vorzugsweise gegebenenfalls durch Chlor im Phenylring substituierte Benzoyloxy- oder Phenacetyloxyhydrine, und Gemische dieser Didrovaltrathydrine mit den entsprechenden Homodidrovaltrathydrinen.

Unter den Valepotriathydrinen, worin A Wasserstoff und B Hydroxy bedeuten, kommen zweckmässigerweise die Hydrine des Isovaleroyloxyhydroxydidrovaltratum (=IVHD) in Frage, z.B. das IVHD-Chlorhydrin.

Sofern X in den erfindungsgemäss verwendeten Valepotriathydrinen für Halogen steht, stellt dieses zweckmässigerweise Chlor, Brom oder Jod, insbesondere Chlor, dar. Ebenso wie die Valepotriathydrine, worin X Halogen darstellt, eignen sich auch Valepotriathydrine, worin X Rhodano, Cyano oder Azido darstellt.

Sofern X in den erfindungsgemäss verwendeten Valepotriathydrinen einen aliphatischen Carbonsäurerest bedeutet, kann der darin enthaltene Alkyl- oder Alkenylrest $R_4$ gerade oder verzweigt sein bis zu 20, vorzugsweise bis zu 10, insbesondere bis zu 6 Kohlenstoffatome enthalten. Als Beispiele geeigneter aliphatischer Carbonsäurereste X seien genannt: Acetoxy, Propionyloxy, Isopropionyloxy, n-Butyryloxy, sek.-Butyryloxy, n-Valeroyloxy, Isovaleroyloxy, 2-Hydroxypropionyloxy, Caprinoyloxy, Lauroyloxy, Stearyloxy, Propargyloxy, Oleyloxy, Cinnamoyloxy.

Sofern X in den erfindungsgemäss verwendeten Valepotriathydrinen einen aromatischen Carbonsäurerest darstellt, kann der in dem Rest $R_4$ enthaltene Phenylrest unsubstituiert oder durch 1 bis 3 Substituenten aus der Gruppe niederes Alkyl, niederes Alkoxy, Hydroxy, Trifluormethyl und Halogen substituiert sein. Unter diesen Substituenten sind insbesondere Halogen, niederes Alkoxy und niederes Alkyl geeignet. Niedere Alkyl- oder Alkoxygruppen können 1 bis 3 Kohlenstoffatome enthalten und insbesondere Methyl oder Methoxy sein. Halogen ist vorzugsweise Chlor.

Die Verbindungen der Formel I besitzen neuartige wertvolle pharmakologische Wirkungen im gastrointestinalen Trakt.

Insbesondere besitzen die Verbindungen überraschenderweise die Fähigkeit, die gastrointestinale Schleimhaut gegenüber verschiedenen Schadwirkungen zu stabilisieren und peptische Ulcusbildung zu hemmen.

Nach Demling [Klin. Gastroenterologie I (1973) Seite 202] und Domschke und Schumpelick [DMW 108, 13 (1983)] liegt bei peptischem Ulcus (ventriculum und duodenum) eine Störung des Gleichgewichtes der auf die Schleimhaut einwirkenden defensiven und aggressiven Faktoren vor. Die Therapie der Ulcuskrankheit muss daher darauf ausgerichtet sein, dieses Gleichgewicht wieder herzustellen.

Ferner besitzen Verbindungen der Formel I die Fähigkeit, Motilitätsstörungen im gastrointestinalen Trakt, insbesondere krampfartigen Störungen der spontanen Motilität im Darmbereich entgegenzuwirken und eine mechanische Überaktivität des Colons zu hemmen.

Die pharmakologischen Eigenschaften der erfindungsgemäss therapeutisch in der Gastroenterologie verwendeten Valepotriathydrine lassen sich in pharmakologischen Standardtests an Tieren nachweisen.

1. Bestimmung der akuten Toxizität

Die akute 7-Tage-Toxizität wird nach einmaliger Applikation per os an der weissen nüchternen NMRI-Maus bestimmt. Die $LD_{50}$ wird als die Dosis definiert, welche einer 50% Mortalität der Tiere am 7. Tag nach der Applikation entspricht. Bei allen untersuchten Substanzen wurde in Dosen bis zu 2150 mg/kg die $LD_{50}$ nicht erreicht.

2. Bestimmung der Hemmwirkung gegen durch Aspirin induzierte Erosion und Ulcusbildung

Bekanntermassen üben die nicht steroidalen Antiphlogistika eine schädigende Wirkung auf die Mucosabarriere aus. Dieser Effekt lässt sich erfindungsgemäss durch Valepotriathydrine weitgehend verhindern, was auf einen therapeutisch protektiven positiven Angriff an der Schleimhaut hinweist.

Testmethode:

Es werden Gruppen von mindestens 6 männlichen Ratten von 180 bis 200 g Körpergewicht pro Testdosis verwendet. Die Prüfsubstanzen werden per os suspendiert in 0,5 ml Suspensionsmedium (2%ige Tyloselösung) pro 100 g Tiergewicht appliziert. Eine Kontrollgruppe von Tieren erhält nur das entsprechende Volumen an Suspensionsmedium. Eine Stunde nach Applikation der Testsubstanzen werden den Tieren per os 200 mg/kg Acetylsalicylsäure ebenfalls suspendiert in 0,5 ml Suspensionsmedium pro 100 g Tiergewicht zur Erzeugung von Ulcera verabreicht. Die Tiere werden fünf Stunden nach der Irritanzapplikation getötet. Anzahl und Grösse der gebildeten Ulcera werden beurteilt. Die Auswertung erfolgt modifiziert nach O. Münchow [Arzneim. Forsch. (drug. Res.) 4, 341–344 (1954)].

Es werden Mittelwert (≙ Ulcus index) und Standardabweichungen berechnet und daraus die Hemmwirkung der Prüfsubstanzen in % gegenüber der Kontrollgruppe bestimmt.

3. Bestimmung der Hemmwirkung auf die spontane Aktivität des Colons in narkotisierten Ratten

Testmethode:

Männliche Ratten von 200–350 g Körpergewicht werden narkotisiert durch i.p. Applikation von 100 mg Xylazin und 50 mg Ketamin/kg als Bolus und anschliessende Dauerinfusion i.p. der gleichen Dosis/Stunde.

Rektum und Colon werden entleert. Die Tiere werden tracheotomiert und laparotomiert und zur Aufrechterhaltung ihrer Körpertemperatur auf einer Wärmeplatte untergebracht. Eine Knopfkanüle, welche an ihrer Spitze mit einem Kunststoffballon (10 ml Durchmesser) versehen ist, wird rektal eingeführt und im Colon plaziert. Nach Füllen mit 3,0 ml Wasser wird der Ballonkatheter mit einem Statham-Druckaufnehmer verbunden. Die Druckänderungen im Colon werden mittels eines Multirecorders kontinuierlich registriert. Gemessen werden die Druckamplituden des Colons in ml Wasser und die Frequenz der Druckänderungen. Zur Bestimmung der Wirkung der Testsubstanzen werden diese in physiologischer Natriumchloridlösung gelöst oder in Tylose suspendiert i.p. oder i.d. appliziert. Die gemittelten Colondruckwerte vor und nach der Applikation der Testsubstanzen werden verglichen und mit Hilfe des t-Tests nach Student ausgewertet.

In den vorstehenden Testmethoden zeigen die Verbindungen der Formel I im allgemeinen befriedigende Resultate in einem Dosisbereich von 20–250 μ mol/kg. Die nachfolgende Tabelle gibt nach den vorstehend beschriebenen Testen erhaltene Ergebnisse wieder. Die für die Testsubstanzen angegebenen Nummern beziehen sich auf die Beispielsnummern der nachstehenden Herstellungsbeispiele.

| Beispiel Nr. | Hemmwirkung auf Aspirin-induzierte Magenulcusbildung in der Ratte | | Wirkung auf die spontane Motilität des Colons in der Ratte | | |
|---|---|---|---|---|---|
| | Dosis in μMol/kg p.o. | % Hemmwirkung | Dosis in μMol/kg | % Änderung der Druckamplituden | % Frequenzänderung |
| 2 | 46.4 | 75 | 46,4 i.d. | −33 | |
| 3 | 46,4 | 65 | 100 i.p. | −48 | |
| 3a | 46,4 | 77 | 100 i.p. | −14 | −19 |
| 5 | 46,4 | 50 | | | |
| 9 | 46,4 | 56 | 100 i.p. | −81 | −25 |
| 10 | 46,4 | 77 | 100 i.p. | −14 | −54 |
| 13 | 46,4 | 76 | 100 i.p. | −42 | −31 |
| 14 | 46,4 | 59 | 100 i.p. | −24 | −55 |
| 15 | 100 | 57 | 100 i.p. | +14 | − 4 |
| 16 | 46,4 | 45 | 100 i.p. | + 4 | −14 |
| 17 | 100 | 59 | 100 i.p. | −72 | −73 |
| 18 | 46,4 | 52 | | | |
| 19 | 100 | 60 | 100 i.p. | −28 | −36 |
| 20 | 46,4 | 64 | 100 i.p. | −28 | −55 |
| 21 | 46,4 | 77 | – | – | – |
| 22 | 100 | 23 | 100 i.p. | −58 | 0 |
| 1 | 21,5 | 64 | 100 i.d. | 0 | −31 |
| 24 | 100 | 53 | – | – | – |

Aufgrund ihrer Wirkungen im gastrointestinalen Trakt sind die Valepotriathydrine der Formel I in der Gastroenterologie als Arzneimittel für grössere Säugetiere und Menschen zur Prophylaxe und Behandlung von peptischen Ulcera und/oder zur Behandlung von krampfartigen Motilitätsstörungen, u.a. Colon irritabele geeignet.

Die zu verwendenden Dosen können individuell verschieden sein und variieren naturgemäss je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch zur Applikation an Menschen und grösseren Säugetieren Arzneiformen mit einem Wirkstoffgehalt von 20–250, insbesondere 25–75 μ mol pro Einzeldosis.

Die Erfindung betrifft ferner neue Valepotriathydrine der Formel I, insbesondere Polyacyloxycyclopenta(c)pyran-Verbindungen der allgemeinen Formel Ia

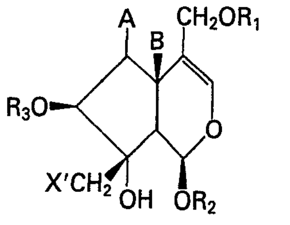

Ia

worin A für Wasserstoff und B für Wasserstoff oder Hydroxy stehen oder A und B gemeinsam eine Bindung bilden, von den Resten $R_1$ bis $R_3$ einer für Isovaleroyl, ein weiterer für Acetyl und der dritte für den Acylrest einer der folgenden Säuren steht: Isovaleriansäure, β-Methylvaleriansäure, α-Isovaleroyloxyisovaleriansäure, α-Acetoxyisovaleriansäure, β-Acetoxyisovaleriansäure, β-Acetoxy-β-methylvaleriansäure oder β-Hydroxyisovaleriansäure, und X' für Cyano, Azido oder einen Acyloxyrest $R_4COO$ steht, worin $R_4$ gegebenenfalls durch Hydroxy substituiertes Äthyl, Propyl, Isopropyl, n-Butyl, 1-Methylpropyl oder tert.-Butyl, eine gegebenenfalls durch Hydroxy substituierte Alkylgruppe mit 5 bis 20 Kohlenstoffatomen, eine Alkenylgruppe mit 3–20 Kohlenstoffatomen oder eine Phenyl-, Phenylalkyl- oder Phenylalkenylgruppe mit bis zu 3 Kohlenstoffatomen in der Alkylen- oder Alkenylenkette, welche gegebenenfalls im Phenylring durch Halogen, Trifluormethyl, Hydroxy, niederes Alkyl oder niederes Alkoxy substituiert sein kann, bedeutet, oder falls B Hydroxy darstellt und/oder $R_2$ nicht Isovaleroyl ist und/oder wenn einer der Reste $R_1$ und $R_3$ Acetyl ist und der andere nicht Isovaleroyl ist, X' auch Halogen, Acetoxy oder Rhodano bedeuten kann, oder A und B gemeinsam eine Bindung bilden, X' Chlor bedeutet und $R_1$ und $R_2$ je Isovaleroyl und $R_3$ Actyl oder $R_1$ Acetyl, $R_2$ Isovaleroyl und $R_3$ β-Acetoxyisovaleroyl bedeuten, sowie deren Herstellung.

Valepotriathydrine der allgemeinen Formel I können auf an sich bekannte Weise aus den entsprechenden Valepotriaten der Formel II hergestellt werden, indem der Epoxidring dieser Valepotriate durch Addition von Säure geöffnet wird.

Hierzu werden die Valepotriate der Formel II in an sich bekannter Weise mit einem Alkalimetallsalz oder einem quartären Ammoniumsalz einer Säure der Formel III

$$H-X \qquad III$$

worin X obige Bedeutung besitzt, in einem unter den Reaktionsbedingungen inerten Lösungsmittel umgesetzt.

Als Alkalimetallsalze der Säuren der Formel III sind insbesondere Natrium- und Kaliumsalze geeignet. Als quartäre Ammoniumsalze eignen sich Tri- oder Tetraalkylammonium- oder Benzyldi- oder -trialkylammoniumsalze, worin die Alkylgruppen niedere Alkylgruppen darstellen, welche vorzugsweise 1 bis 4 Kohlenstoffatome enthalten, oder auch Salze von cyclischen Aminen, beispielsweise Pyridinium-, Pyrrolidinium- oder N-Niederalkylpyrrolidiniumsalze. Als Beispiele geeigneter quartärer Ammoniumsalze seien genannt Tetrabutylammonium-, Triäthylammonium-, Tetramethylammonium-, Benzyltriäthylammonium- oder Pyridiniumsalze. Quartäre Ammoniumsalze der Säuren der Formel III können gewünschtenfalls in situ aus den entsprechenden Aminen gebildet werden.

Als inerte Lösungsmittel eignen sich beispielsweise halogenierte Kohlenwasserstoffe, wie Methylenchlorid oder Chloroform, aromatische Kohlenwasserstoffe, wie Benzol oder Toluol, Acetonitril, Eisessig, oder niedere Alkohole oder Gemische dieser Lösungsmittel. Falls Alkalimetallsalze eingesetzt werden, kann die Reaktionslösung auch aus einem Gemisch der vorgenannten Lösungsmittel mit Wasser bestehen, beispielsweise niedere Alkohole/Wasser oder Eisessig/Wasser. Falls wasserhaltige Lösungsmittel und/oder Alkalimetallsalze der Säuren der Formel III verwendet werden, kann es vorteilhaft sein, zur Abpufferung des Reaktionsgemisches auf einen pH-Bereich von 3 bis 7 Puffersalze, z.B. Natriumacetat oder Ammoniumchlorid zuzusetzen. Zweckmässigerweise kann dem Reaktionsgemisch eine zum Abpuffern der bei der Umsetzung freiwerdenden Base ausreichende Menge einer unter den Reaktionsbedingungen inerten Säure oder der Säure der Formel III zugesetzt werden. Falls die Säure der Formel III eine organische Carbonsäure ist, kann ein Überschuss dieser Säure als Lösungsmittel dienen. Zum Beispiel kann die Reaktion so durchgeführt werden, dass das Valepotriat in der Säure gelöst wird und durch Zugabe eines tertiären Amines ein quartäres Ammoniumsalz der Säure in situ gebildet wird.

Es kann sich als zweckmässig erweisen, insbesondere wenn Alkalimetallsalze der Säuren der Formel III eingesetzt werden, ein als Phasentransfer-Katalysator geeignetes anorganisches Tetraalkylammoniumsalz oder Tetraalkylphosphoniumsalz, z.B. ein Halogenid oder ein Hydrogensulfat, zusetzen. Als Tetraalkylammoniumsalz eignen sich vorzugsweise Niederalkylammoniumsalze, in welchen die Alkylgruppen beispielsweise 2 bis 4 Kohlenstoffatome enthalten. In den Tetraalkylphosphoniumsalzen können Alkylgruppen mit 1 bis 20 Kohlenstoffatomen enthalten sein. Als Beispiele von als Katalysator geeigneten Salzen seien genannt Tetrabutylammoniumhydrogensulfat, Tetrabutylammoniumchlorid, Hexadecyltributylphosphoniumbromid.

Die Reaktionstemperatur kann je nach Reaktivität des einzuführenden Säurerestes X zwischen 0 und 120 °C betragen, und die Reaktionsdauer kann zwischen einer halben und 24 Stunden variieren.

Die Valepotriathydrine der Formel I bzw. deren Gemische können auf an sich bekannte Weise aus dem Reaktionsgemisch isoliert werden.

Im allgemeinen werden als Ausgangssubstanzen der Formel II aus natürlichen Pflanzenextrakten gewonnene Valepotriatgemische verwendet, welche neben dem jeweiligen Hauptvalepotriat noch untergeordnete Mengen dazu isomerer oder nahe verwandter Valepotriate, welche in der Pflanze gemeinsam mit dem Hauptvalepotriat vorkommen, enthalten. So enthalten die verwendeten Valepotriate der Formel II vielfach Beimengungen der entsprechenden Homoverbindungen der Formel II, worin ein Isovaleroyloxyrest durch einen Methylvaleroyloxyrest ersetzt ist. Das in dem Ausgangsmaterial der Formel II vorhandene Verhältnis von Hauptkomponente zur entsprechenden Homoverbindung bleibt bei der Umsetzung erhalten und tritt auch in dem Endprodukt der Formel I auf. Das Verhältnis von Hauptkomponente zu der entsprechenden Homoverbindung und allfälligen weiteren Isomeren kann in dem Ausgangsprodukt und/oder dem Endprodukt auf an sich bekannte Weise durch $^{13}C$-NMR-Spektroskopie bestimmt werden.

Gewünschtenfalls können jedoch isomerenreine Verbindungen sowohl auf der Stufe der Ausgangsvalepotriate der Formel II als auch auf der Stufe der Valepotriathydrine der Formel I aus etwaigen Gemischen auf an sich bekannte Weise durch Säulenchromatographie, vorzugsweise Hochdruckflüssigkeitssäulenchromatographie, abgetrennt werden.

Die Verbindungen der Formel I können erfindungsgemäss zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate, wie Kapseln, Tabletten, Granulate oder Dragées, genannt oder auch Suppositorien. Feste Präparate können pharmazeutisch übliche anorganische und/oder organische Trägerstoffe, wie z.B. Talkum, Milchzucker oder Stärke neben pharmazeutisch üblichen Hilfsmitteln, beispielsweise Gleitmitteln wie Magnesiumstearat oder Tablettensprengmitteln enthalten. Flüssige Präparate wie Lösungen, Suspensionen oder Emulsionen können die üblichen Verdünnungsmittel, wie Wasser, Öle, z.B. Triglyceridgemische von gesättigten Pflanzenfettsäuren, oder Vaseline® und/oder Suspensionsmittel, wie Polyäthylenglykole und dergleichen enthalten. Es können zusätzlich weitere Hilfsstoffe zugegeben werden, wie z.B. Konservierungsmittel, Stabilisierungsmittel, Geschmackskorrigenzien und dergleichen.

Gewünschtenfalls können feste orale Arzneiformen auch die Freisetzung des Wirkstoffes verzögernde Stoffe, wie z.B. Polyvinylacetat, Acrylat- oder Methacrylatcopolymere, höhere Fettalkohole und andere wachsartige Substanzen, enthalten.

Die Wirkstoffe können mit den pharmazeutischen Hilfs- und/oder Trägerstoffen in an sich bekannter Weise gemischt und formuliert werden. Gewünschtenfalls können die Wirkstoffe zunächst in an sich bekannter Weise, z.B. nach der in DE-OS 2849029 beschriebenen Methode, mikroverkapselt werden. Zur Herstellung fester Arzneiformen können die Wirkstoffe, gegebenenfalls in mikroverkapselter Form, beispielsweise mit den Hilfs- und/oder Trägerstoffen in üblicher Weise gemischt und nass oder trocken granuliert werden. Je nach Art der verwendeten Zusatzstoffe kann gegebenenfalls auch durch einfaches Mischen ein direkt tablettierbares Pulver erhalten werden.

Das Granulat oder Pulver kann direkt in Kapseln abgefüllt oder in üblicher Weise zu Tablettenkernen verpresst werden. Diese können gewünschtenfalls in bekannter Weise dragiert werden. Gewünschtenfalls können Tabletten, Dragées und Kapseln auf an sich bekannte Weise mit einem magensaftresistenten Überzug versehen werden.

Gewünschtenfalls können die Valepotriathydrine auch in einem flüssigen Trägerstoff suspendiert oder gelöst in Weichgelatinekapseln abgefüllt werden.

Die nachfolgenden Beispiele erläutern die Erfindung, sollen deren Umfang jedoch in keiner Weise beschränken.

Beispiel 1

Isovaltratchlorhydrin

25 g Isovaltratum (etwa 10% Valtratum enthaltend) werden in 100 ml Methylenchlorid gelöst und die Lösung mit 40 g Tetrabutylammoniumchlorid versetzt. Das Reaktionsgemisch wird ca. 6h lang auf 60 °C erwärmt.

Anschliessend wird zur Aufarbeitung mit Wasser verdünnt und mit Äther extrahiert. Die organische Phase wird einmal mit Wasser gewaschen, über Natriumsulfat getrocknet, filtriert und im Vakuum zur Trockene eingeengt. Das als Rückstand verbleibende Rohprodukt (ca. 30 g) wird durch Säulenchromatographie über Aluminiumoxid unter Verwendung von n-Hexan, enthaltend 5–10% 2-Butanon als Elutionsmittel, gereinigt. Es werden 22,97 g gereinigtes Rohprodukt erhalten, welches neben Isovaltratchlorhydrin auch Valtratchlorhydrin enthält. Nach mehrmaligem Umkristallisieren aus n-Hexan/Äther werden 7,8 g reines Isovaltratchlorhydrin erhalten.

Fp.: 78–79 °C

$[\alpha]_D^{20} = +187{,}5°$ (c = 1 in $CH_2Cl_2$)

Beispiel 2

Didrovaltratisovaleroyloxyhydrin

60 g Didrovaltratum (enthaltend ~20% Homodidrovaltratum) werden in 135 ml Isovaleriansäure gelöst und die Lösung mit 24 ml Triäthylamin und 21 g Tetrabutylammoniumhydrogensulfat versetzt. Das Reaktionsgemisch wird 14 h lang auf 60 °C erwärmt. Anschliessend wird zur Aufarbeitung vorsichtig auf Eiswasser gegossen, das Gemisch schonend mit Natriumbicarbonatlösung neutralisiert und mit Äther extrahiert. Die organische Phase wird über Natriumsulfat getrocknet, filtriert und im Vakuum bis zur Trockne eingeengt. Das verbleibende Rohprodukt wird chromatographisch über 250 g Kieselgel unter Verwendung von n-Hexan/Äther als Elutionsmittel gereinigt. Es werden 91 g Rohprodukt erhalten, welches noch Isovaleriansäure enthält. Die Reinigung über Kieselgel wird ein zweites Mal wiederholt. Es werden

65,81 g gereinigtes Produkt erhalten. Dieses wird in Äther aufgenommen, einmal mit Wasser, einmal mit Natriumbicarbonatlösung und wiederum mit Wasser ausgeschüttelt. Anschliessend wird die Ätherphase über Natriumsulfat getrockriet, mit Kohle geklärt, filtriert und im Vakuum bis zur Trockne eingeengt. Es werden 54,46 g öliges Didrovaltratisovaleroyloxyhydrin (enthaltend ~20% Homodidrovaltratisovaleroyloxyhydrin) erhalten.

$[\alpha]_D^{20} = -19°$ (c = 1 in $CH_3OH$)

Beispiel 3

Didrovaltratchlorhydrin (homodidrovaltratchlorhydrinhaltig)

26 g Didrovaltratum (enthaltend ca. 20% Homodidrovaltratum) werden in 100 ml Acetonitril gelöst und die Lösung mit 56 g Benzyltriäthylammoniumchlorid und 13 ml Eisessig versetzt und ca. 4 h lang auf 60 °C erhitzt. Anschliessend wird das Reaktionsgemisch zur Aufarbeitung abgekühlt, mit Wasser verdünnt und mit Äther extrahiert. Die Ätherphase wird dreimal mit Wasser gewaschen, über Natriumsulfat getrocknet, filtriert und im Vakuum bis zur Trockne eingeengt. Das als Rückstand verbleibende braune Rohprodukt wird über Kieselgel unter Verwendung von n-Hexan, enthaltend bis zu 20% 2-Butanon als Elutionsmittel gereinigt. Das Eluat wird zur Trockne eingeengt, der Rückstand in Äther aufgenommen, die ätherische Lösung mit Natriumbicarbonatlösung und anschliessend dreimal mit Wasser gewaschen, über Natriumsulfat getrocknet, mit Kohle geklärt, filtriert und im Vakuum bis zur Trockne eingeengt. Es werden 20,6 g öliges Didrovaltratchlorhydrin (enthaltend ~20% Homodidrovaltratchlorhydrin) erhalten.

$[\alpha]_D^{20} = -16{,}8°$ (c = 1 in $CH_3OH$)

Beispiel 3a

Didrovaltratchlorhydrin (rein)

A) Herstellung von reinem Didrovaltratumausgangsprodukt

500 g Aluminiumoxid werden in 2450 ml Petroläther und 50 ml Eisessig aufgeschlämmt, in eine Säule gegeben und mit n-Heptan säurefrei gewaschen. 5 g rohes Didrovaltratum (enthaltend ca. 15% Homodidrovaltratum und 5% Isovaltratum) werden auf die Säule gegeben und mit n-Heptan fraktioniert eluiert.

Die nur Didrovaltratum enthaltenden Fraktionen werden im Vakuum eingeengt und das als Rückstand verbleibende Didrovaltratum wird aus Äther/n-Hexan umkristallisiert. Schmelzpunkt 63–64 °C, Ausbeute 1,6 g.

Aus den weitgehend didrovaltratumfreien Fraktionen wird beim Einengen als Rückstand rohes Homodidrovaltratum (enthaltend ca. 10% Isovaltratum) erhalten.

B) Herstellung des Didrovaltratchlorhydrins

0,82 g Didrovaltratum werden in 20 ml Methylenchlorid gelöst und die Lösung mit 0,9 g Benzyltriäthylammoniumchlorid und 0,4 g Maleinsäure versetzt. Das Reaktionsgemisch wird 24 h bei Raumtemperatur gerührt. Dann wird zweimal mit Wasser ausgeschüttelt und die organische Phase über Natriumsulfat getrocknet, filtriert und im Vakuum eingeengt. Der verbleibende Rückstand wird chromatographisch über Kieselgel unter Verwendung von n -Hexan/2-Butanon als Elutionsmittel gereinigt. Es werden 0,82 g (entsprechend einer Ausbeute von 92%) reines Didrovaltratchlorhydrin als Öl erhalten.

$[\alpha]_D^{20} = -20{,}6°$ (c = 1 in $CH_3OH$)

Beispiel 3b

Homodidrovaltratchlorhydrin (rein)

0,8 g rohes Homodidrovaltratum (enthaltend ca. 10% Isovaltratum, hergestellt nach Beispiel 3a A) wird wie in Beispiel 3a in das entsprechende Chlorhydrin überführt. Aus dem Rohprodukt wird durch Hochdruckflüssigkeitschromatographie (HPLC) unter Verwendung von Kieselgel mit einer Korngrösse von 0,007 ml (Handelsprodukt Lichrosorp der Fa. Merck) unter Verwendung von n-Hexan/Äthanol 99,5:0,5 als Elutionsmittel reines Homodidrovaltratchlorhydrin isoliert.

$[\alpha]_D^{20} = -20{,}6°$ (c = 1 in $CH_3OH$)

Beispiel 4

Didrovaltratazidohydrin

2,12 g Didrovaltratum (enthaltend ~20% Homodidrovaltratum) werden in 50 ml Methanol und 10 ml Wasser gelöst und die Lösung mit 6,65 g Natriumazid und 5,53 g Ammoniumchlorid versetzt. Das Reaktionsgemisch wird bei Raumtemperatur ca. 12 h stehengelassen. Anschliessend wird zur Aufarbeitung mit Eiswasser verdünnt und mit Äther extrahiert. Die Ätherphase wird über Natriumsulfat getrocknet, über Kohle geklärt, filtriert und im Vakuum bis zur Trockne eingeengt. Es werden 1,66 g öliges Didrovaltratazidohydrin (enthaltend ~20% Homodidrovaltratazidohydrin) erhalten (entsprechend einer Ausbeute von 71%).

$[\alpha]_D^{20} = -38{,}6°$ (c = 1 in $CH_3OH$)

Beispiel 5

Didrovaltratacetoxyhydrin

10 g Didrovaltratum (enthaltend ~20% Homodidrovaltratum) werden in 11 ml Eisessig und 1,1 ml Essigsäureanhydrid bei 80 °C gelöst. Der Ansatz wird auf 20 °C abgekühlt und mit 25 ml Triäthylamin versetzt. Die Lösung wird anschliessend 2,5 h auf 80 °C erhitzt. Dann wird die Lösung zur Aufarbeitung auf Eiswasser gegeben und mit Äther extrahiert. Die Ätherextrakte werden über Natriumsulfat getrocknet, filtriert und im Vakuum eingeengt. Es werden 9,8 g Didrovaltratacetoxyhydrin (enthaltend ~20% Homodidrovaltratacetoxyhydrin) erhalten (entsprechend 85,9% Ausbeute erhalten).

$[\alpha]_D^{20} = -38{,}0°$ (c = 1 in $CH_3OH$)

Beispiel 6

Valtratrhodanohydrin

6,0 g Valtratum und 12 g Natriumacetat werden in 60 ml Eisessig gelöst und mit einer Lösung von 2,775 Kaliumrhodanid in 14 ml Wasser versetzt. Die Reaktionsmischung wird 12 h bei einer Temperatur zwischen 0 und 5 °C stehengelassen. Anschliessend wird der Ansatz zur Aufarbeitung mit der doppelten Menge Wasser versetzt und 4 mal mit je 50 ml Äther extrahiert. Die vereinigten Ätherphasen werden mit Wasser gewaschen, über Magnesiumsulfat mit Kohlezusatz getrocknet, filtriert und im Vakuum eingeengt. Es wird ein farbloses Öl erhalten, das sehr rasch kristallisiert. Nach Umkristallisation aus Äther/Benzin wird Valtratrhodanohydrin mit einer Ausbeute von 75% erhalten.

Fp.: 106–109 °C

$[\alpha]_D^{20} = +209,17°$ (c = 1 in $CH_3OH$)

Beispiel 7

Valtratbromhydrin

2,5 g Valtratum und 2 g Natriumacetat werden in 10 ml Eisessig gelöst und mit einer Lösung von 0,25 g Natriumbromid in 2 ml Wasser versetzt. Der Ansatz wird 2 Tage bei 22 °C stehengelassen. Anschliessend wird zur Aufarbeitung mit Wasser verdünnt, mit Äther/Benzin 1:1 extrahiert und die organische Phase mit Wasser säurefrei gewaschen, über Magnesiumsulfat mit Kohlezusatz getrocknet, filtriert und eingeengt. Der zunächst ölige Rückstand kristallisiert nach Verreiben mit wenig Äther/Benzin. Nach Umkristallisieren aus Äther/Benzin wird das Valtratbromhydrin mit einem Schmelzpunkt von 66–68 °C erhalten.

Beispiel 8

Valtratjodhydrin

5 g Valtratum und 10 g Natriumacetat werden in 50 ml Eisessig gelöst, und diese Lösung wird mit einer Lösung von 1,785 g Natriumjodid in 8 ml Wasser versetzt. Nach 4 h Stehen bei 0 bis 5 °C wird das zu einem Kristallbrei erstarrte Gemisch mit Eiswasser verdünnt und das Valtratjodhydrin abfiltriert, mit Wasser gewaschen und im Vakuum bei 60 °C getrocknet. Die Mutterlaugen werden nochmals mit Äther extrahiert, die Ätherphase getrocknet, gewaschen und eingeengt, wobei nochmals Valtratjodhydrin als Rückstand erhalten wird. Das vereinigte Valtratjodhydrin wird aus Äther/n-Heptan umkristallisiert. Es werden 5,58 g (entsprechend 86% Ausbeute) mit einem Schmelzpunkt von 112 °C erhalten.

$[d]_D^{20} = +217,1°$ (c = 1 in $CH_3OH$)

Beispiel 9

Didrovaltratbenzoyloxyhydrin

12,6 g einer 25%igen methanolischen Lösung von Tetrabutylammoniumhydroxid werden mit 3 g Benzoesäure versetzt. Die Lösung wird eingeengt und das als Rückstand verbleibende Tetrabutylammoniumbenzoat wird in 12 ml Acetonitril gelöst. Zu der Lösung werden 2 g Didrovaltratum (enthaltend ~15% Homodidrovaltratum) gegeben und das Reaktionsgemisch wird 4 h bei 80°C gerührt. Zur Aufarbeitung wird die abgekühlte Lösung im Vakuum eingeengt. Das als Rückstand verbleibende Rohprodukt wird über Kieselgel unter Verwendung von Methylenchlorid, enthaltend bis zu 6% Äther als Elutionsmittel gereinigt. Es werden aus den verschiedenen Eluatfraktionen 500 mg noch leicht verunreinigtes Produkt und 2,08 g dünnschichtchromatographisch reines Produkt als Öl erhalten. Das zunächst ölige Didrovaltratbenzoyloxyhydrin (enthaltend ~15% Homodidrovaltratbenzoyloxyhydrin) kristallisiert beim Stehen aus. Nach Umkristallisation aus Hexan/Äther besitzt das Produkt einen Schmelzpunkt von 85 bis 86 °C.

Ausbeute 80,6%.

$[\alpha]_D^{20} = -22,9°$ (c = 1 in $CH_3OH$)

Beispiel 10

Didrovaltrat(2-chlorphenyl)acetoxyhydrin

6,4 g einer 25%igen methanolischen Tetrabutylammoniumhydroxidlösung werden mit 2,1 g 2-Chlorphenylessigsäure versetzt und die Lösung im Vakuum eingeengt. Das im Rückstand verbleibende Tetrabutylammonium-(2-chlorphenyl)acetat wird in 6 ml Acetonitril gelöst und die Lösung mit 1 g Didrovaltratum (enthaltend ~20% Homodidrovaltratum) versetzt und 2 bis 3 h bei 80 °C gerührt. Zur Aufarbeitung wird die Lösung im Vakuum eingeengt. Das als Rückstand verbleibende Rohprodukt wird chromatographisch über eine Kieselgelsäule unter Verwendung von Methylenchlorid, enthaltend bis zu 10% Äther als Elutionsmittel gereinigt.

Aus den Eluatfraktionen werden 700 mg gereinigtes Didrovaltrat(2-chlorphenyl)acetoxyhydrin (enthaltend ~15% Homoverbindung) als Öl erhalten.

$[d]_D^{20} = -24,4°$ (c = 1 in $CH_3OH$)

Beispiel 11

Isovaleroyloxyhydroxydidrovaltratchlorhydrin (= JVHD-Chlorhydrin)

22 g rohes Isovaleroyloxyhydroxydidrovaltrat (enthaltend Beimengungen von anderen Valepotriaten) werden in 100 ml Acetonitril gelöst. Die Lösung wird mit 22 g Benzyltriäthylammoniumchlorid versetzt und das Reaktionsgemisch 8 h bei 60°C gerührt. Anschliessend werden 0,5 ml Eisessig zugegeben und nochmals 2 h bei 60 °C gerührt. Zur Aufarbeitung wird das Reaktionsgemisch mit Eiswasser zersetzt und mit Äther extrahiert. Die organische Phase wird über Natriumsulfat getrocknet, filtriert und im Vakuum bis zur Trockne eingeengt. Das als Rückstand erhaltene Rohprodukt wird chromatographisch über Kieselgel unter Verwendung von n-Hexan, enthaltend 2-Butanon als Elutionsmittel, gereinigt. Es werden 15,96 g Rohprodukt erhalten. Dieses wird in Äther gelöst und einmal mit Natriumbicarbonatlösung und einmal mit Wasser ausgeschüttelt. Anschliessend wird die Ätherphase über Natriumsulfat getrocknet und bis zur Trockne eingeengt. Es werden 2 g Rohkristallisat IVHD-Chlorhydrin erhalten. Nach mehrmaligem Umkristallisieren aus n-Hexan/Äther werden 1,14 g Isovaleroyloxyhydroxydidrovaltratchlorhydrin mit einem Schmelzpunkt von 80°C erhalten.

$[\alpha]_D^{20} = -55,1°$ (c = 1 in $CH_3OH$)

Beispiel 12

Desacetyl-11-β-hydroxyisovaleroyloxyvaltratjodhydrin

8 g eines Gemisches aus Desacetyl-11-β-hydroxyisovaleroylvaltratum mit Isovaleroyloxyhydroxydidrovaltratum, Didrovaltratum, Homodidrovaltratum und 1-α-Acevaltratum werden zusammen mit 16 g Natriumacetat in 100 ml Eisessig gelöst und die Lösung mit 5 g Natriumjodid in 20 ml Wasser versetzt. Das Reaktionsgemisch wird 2h bei Raumtemperatur gerührt und anschliessend mit 300 ml Wasser verdünnt und mit Äther extrahiert. Die organische Phase wird über Natriumsulfat getrocknet, filtriert und im Vakuum eingeengt. Aus dem erhaltenen rohen Jodhydringemisch wird die Titelverbindung chromatographisch abgetrennt.

Zur Herstellung der für die chromatographischen Trennung benötigten Säule werden 400 g Aluminiumoxid in 500 ml n-Hexan und 40 ml Eisessig aufgeschlämmt, in eine Säule gefüllt und mit n-Hexan säurefrei gewaschen. Das zu trennende Jodhydringemisch wird auf diese Säule gegeben und mit n-Hexan, welches steigende Mengen an 2-Butanon enthält, eluiert. Durch Änderung des 2-Butanon-Zusatzes in dem Elutionsmittel können einzelne Jodhydrine fraktioniert aus dem Gemisch eluiert werden. Nach Elution der übrigen Jodhydrine mit n-Hexan, welches steigende Mengen von bis zu 50% an 2-Butanon enthält, wird das Desacetyl-11-β-hydroxyisovaleroylvaltratjodhydrin mit 100%igem 2-Butanon von der Säule eluiert. Aus dem Eluat werden 0,71 g Desacetyl-11-β-hydroxyisovaleroylvaltratjodhydrin (enthaltend 30% entsprechende Homoverbindungen) erhalten.

Analog den vorstehend beschriebenen Beispielen werden auch die folgenden Valepotriathydrine hergestellt:

| Beispiel Nr. | Substanzname | $[\alpha]_D^{20}$ (c = 1, $CH_3OH$) | Bemerkungen (H: = Gehalt an Homoverbindung nach NMR-Spektrum) |
|---|---|---|---|
| 13 | Didrovaltrat-(4-methylbenzoyl)oxyhydrin | −23,5° | H: 10% Fp 76–78 °C |
| 14 | Didrovaltrat-(2-chlorbenzoyl)oxyhydrin | −23,2° | H: 20% Fp 100–101 °C |
| 15 | Didrovaltrat-(3-trifluormethylbenzoyl)-oxyhydrin | −12,4° | H: 15% |
| 16 | Didrovaltrat-(3-methoxybenzoyl)oxyhydrin | −11,6° | H: 15% |
| 17 | Didrovaltrat-(3,4-dimethoxybenzoyl)-oxyhydrin | − 8,5° | H: 15% |
| 18 | Didrovaltrat-(3,4,5-trimethoxyphenyl)-acetoxyhydrin | −14,0° | H: 20% |
| 19 | Didrovaltrat-(2-methoxyphenyl)acetoxyhydrin | −34,5° | H: 15% Fp 93–94 °C |
| 20 | Didrovaltrat-(4-fluorphenyl)acetoxyhydrin | −23,0° | H: 15% |
| 21 | Didrovaltratcinnamoyloxyhydrin | −31,7° | H: 20% |
| 22 | Didrovaltratcaprinoyloxyhydrin | −28,6° | H: 10% |
| 23 | Didrovaltratoleyloxyhydrin | −21,1° | H: 20% |
| 24 | Didrovaltrat-(4-hydroxybenzoyl)oxyhydrin | − 2,4° | H: 20% |
| 25 | Didrovaltrat-(3,4-dihydroxybenzoyl)oxyhydrin | −13,9° | H: 20% |
| 26 | Didrovaltrat-(2-hydroxybenzoyl)oxyhydrin | − 9,3° | H: 15% |

Beispiel I
Didrovaltratchlorhydrinhaltige Tabletten.

Man stellt Tabletten in folgender Zusammensetzung pro Tablette her:

| | |
|---|---|
| Didrovaltratchlorhydrin (enthaltend ~20% Homodidrovaltratchlorhydrin) | 25 mg |
| mikrokristalline Zellulose (Avicel® PH 101) | 78 mg |
| hochdisperse Kieselsäure (Aerosil® R 972) | 12,5 mg |
| Carboxymethylzellulose (Tylose®) | 7 mg |

Der Wirkstoff wird in Methylenchlorid gelöst. Die mikrokristalline Zellulose und die hochdisperse Kieselsäure werden gemischt und mit der Wirkstofflösung verrieben. Die erhaltene Verreibung wird getrocknet und mit einer wässrigen Tyloselösung befeuchtet. Das erhaltene feuchte Granulat wird durch ein Sieb mit 2 mm Maschenweite gepresst, im Wirbelschichttrockner bei 40–45 °C getrocknet und nochmals durch ein Sieb mit 1,5 mm Maschenweite gegeben, und anschliessend in einem Mixer mit folgenden weiteren Hilfsstoffen vermischt:

| | |
|---|---|
| Quervernetztes Polyvinylpyrrolidon (crospovidone USP 20/NF 15 3) | 4,5 mg |
| Magnesiumstearat | 1,5 mg |
| hochdisperse Kieselsäure | 1,5 mg |

und sodann zu Tabletten von 130 mg verpresst.

Beispiel II
Didrovaltratbenzoyloxyhydrinhaltige Kapseln.

Man stellt Kapseln mit folgender Zusammensetzung her:

| | |
|---|---|
| Didrovaltratbenzoyloxyhydrin | 10 mg |
| Mannit | 175 mg |
| Talkum | 12 mg |
| kolloidale Kieselsäure | 1 mg |
| Magnesiumstearat | 2 mg |
| | 200 mg |

Die gesiebten Substanzen werden gemischt und das erhaltene Pulver in Portionen von 200 mg in Kapseln abgefüllt.

Beispiel III
Didrovaltratisovaleroyloxyhydrinhaltige Weichgelatinekapseln

Zusammensetzung:

| | |
|---|---|
| Didrovaltratisovaleroyloxyhydrin (enthaltend ~20% Homodidrovaltrat-isovaleroyloxyhydrin) | 30 Gew.-Teile |
| Miglyol 812®** | 278 Gew.-Teile |
| Gesamt | 308 Gew.-Teile |

** = öliges Triglyceridgemisch von gesättigten Pflanzenfettsäuren mit C8-, C10- und C12-Kettenlänge. Hersteller: Dynamit Nobel AG.

Herstellung:

Der Wirkstoff wird unter leichtem Erwärmen und Rühren in dem Miglyol 812 aufgelöst. Die Lösung wird zu im Mittel 302 Mikroliter Lösung enthaltenen Weichgelatinekapseln verarbeitet. Valepotriatwirkstoffgehalt pro Kapsel 30 mg.

Beispiel IV

Didrovaltrat-(2-chlorphenyl)acetoxyhydrinhaltige Suppositorien

Man stellt Suppositorien in folgender Zusammensetzung pro Suppositorium her:

| | |
|---|---|
| Didrovaltrat-(2-chlorphenyl)acetoxyhydrin (enthaltend ~20% Homodidrovaltrat-(2-chlorphenyl)acetoxyhydrin) | 20 mg |
| Kakaobutter | 1980 mg |

Der Wirkstoff und die feingeriebene Suppositoriengrundmasse werden zusammengeschmolzen und gründlich vermischt. Aus der durch Rühren homogen gehaltenen Schmelze werden Suppositorien von 2 g gegossen.

Beispiel V

Isovaltratchlorhydrinhaltige Kapseln

Man stellt Kapseln mit folgender Zusammensetzung her:

| | |
|---|---|
| Isovaltratchlorhydrin | 20 mg |
| Mannit | 165 mg |
| Talkum | 12 mg |
| kolloidale Kieselsäure | 1 mg |
| Magnesiumstearat | 2 mg |
| | 200 mg |

Die gesiebten Substanzen werden gemischt und das erhaltene Pulver in Portionen von 200 mg in Kapseln abgefüllt.

**Patentansprüche für die Vertragsstaaten BE, CH, DE, FR, GB, IT, NL**

1. Verwendung von Polyacyloxycyclopenta(c)-pyran-Verbindungen der allgemeinen Formel I

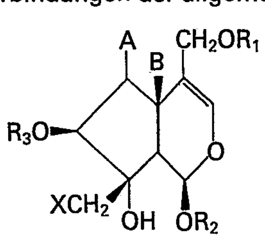

I

worin A für Wasserstoff und B für Wasserstoff oder Hydroxy stehen oder A und B gemeinsam eine Bindung bilden, von den Resten $R_1$ bis $R_3$ einer für Isovaleroyl, ein weiterer für Acetyl und der dritte für den Acylrest einer der folgenden Säuren steht: Isovaleriansäure, β-Methylvaleriansäure, α-Isovaleroyloxyisovaleriansäure, α-Acetoxyisovaleriansäure, β-Acetoxyisovaleriansäure, β-Acetoxy-β-methylvaleriansäure oder β-Hydroxyisovaleriansäure, und X für Halogen, Cyano, Rhodano, Azido oder einen Acyloxyrest $R_4COO$ steht, worin $R_4$ eine gegebenenfalls durch Hydroxy substituierte Alkylgruppe mit 1 bis 20 Kohlenstoffatomen oder eine Alkenylgruppe mit 3 bis 20 Kohlenstoffatomen oder eine Phenyl-, Phenylalkyl- oder Phenylalkenylgruppe mit bis zu 3 Kohlenstoffatomen in der Alkylen- oder Alkenylenkette, welche gegebenenfalls im Phenylring durch Halogen, Trifluormethyl, Hydroxy, niederes Alkyl oder niederes Alkoxy substituiert sein kann, bedeutet, zur Herstellung von pharmazeutischen Zubereitungen zur Behandlung und Prophylaxe von peptischen Ulcera und/oder zur Behandlung von krampfartigen Motilitätsstörungen im gastrointestinalen Trakt in grösseren Säugetieren und Menschen.

2. Verwendung von Polyacyloxycyclopenta(c)-pyran-Verbindungen gemäss Anspruch 1, dadurch gekennzeichnet, dass Polyacyloxycyclopenta(c)pyran-Verbindungen der Formel I verwendet werden, worin $R_1$, $R_2$, $R_3$, A und B die in Anspruch 1 angegebene Bedeutung besitzen und X Halogen, Alkanoyloxy mit 2 bis 10 Kohlenstoffatomen oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

3. Verwendung von Polyacyloxycyclopenta(c)-pyran-Verbindungen gemäss Anspruch 1 oder 2, dadurch gekennzeichnet, dass Polyacyloxycyclopenta(c)pyran-Verbindungen der Formel I verwendet werden, worin A, B, $R_1$, $R_3$ und X die in Anspruch 1 oder 2 angegebenen Bedeutung besitzen und $R_2$ Isovaleroyl oder β-Methylvaleroyl bedeutet.

4, Verwendung von Polyacyloxycyclopenta(c)-pyran-Verbindungen gemäss Anspruch 3, dadurch gekennzeichnet, dass Polyacyloxycyclopenta(c)pyran-Verbindungen verwendet werden, worin A, B, X und $R_2$ die in Anspruch 3 angegebene Bedeutung besitzen und von den Resten $R_1$ und $R_3$ einer für Acetyl und der andere für Isovaleroyl oder β-Acetoxyisovaleroyl oder, falls B = OH, auch einer für α-Isovaleroyloxyisovaleroyl und der andere für Acetyl steht.

5. Verwendung von Polyacyloxycyclopenta(c)-pyran-Verbindungen gemäss Anspruch 1 oder 2, dadurch gekennzeichnet, dass Polyacyloxycyclopenta(c)pyran-Verbindungen der Formel I verwendet werden, worin A und B je Wasserstoff bedeuten und $R_1$, $R_2$, $R_3$ und X die in Anspruch 1 oder 2 angegebene Bedeutung besitzen.

6. Verwendung von Polyacyloxycyclopenta(c)-pyran-Verbindungen gemäss Anspruch 1, da-

durch gekennzeichnet, dass Polyacyloxycyclopenta(c)pyran-Verbindungen der Formel I verwendet werden, worin A und B je Wasserstoff bedeuten, $R_1$ Isovaleroyl, $R_2$ Isovaleroyl oder β-Methylvaleroyl und $R_3$ Acetyl bedeuten und X die in Anspruch 1 angegebene Bedeutung besitzt.

7. Verwendung von Polyacyloxycyclopenta(c)-pyran-Verbindungen gemäss Anspruch 6, dadurch gekennzeichnet, dass Hydrine des Didrovaltratum und/oder des Homodidrovaltratum verwendet werden, worin X Halogen, Alkanoyloxy mit 2 bis 10 Kohlenstoffatomen oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

8. Verwendung von Polyacyloxycyclopenta(c)-pyran-Verbindungen gemäss Anspruch 7, dadurch gekennzeichnet, dass Didrovaltratchlorhydrin und/oder Homodidrovaltratchlorhydrin verwendet werden.

9. Verwendung von Polyacyloxycyclopenta(c)-pyran-Verbindungen gemäss Anspruch 7, dadurch gekennzeichnet, dass Didrovaltratalkanoyloxyhydrine und/oder Homodidrovaltratalkanoyloxyhydrine mit einem Alkanoyloxyrest mit 2 bis 7 Kohlenstoffatomen verwendet werden.

10. Verwendung von Polyacyloxycyclopenta(c)-pyran-Verbindungen gemäss Anspruch 9, worin der Alkanoyloxyrest Isovaleroyloxy darstellt.

11. Verwendung von Polyacyloxycyclcopenta(c)-pyran-Verbindungen gemäss Anspruch 7, dadurch gekennzeichnet, dass Hydrine des Didrovaltratum und/oder des Homodidrovaltratum verwendet werden, worin X eine Benzoyloxy- oder Phenylacetoxygruppe bedeutet, deren Phenylring gegebenenfalls durch Chlor substituiert sein kann.

12. Verwendung von Polyacyloxycyclopenta(c)-pyran-Verbindungen gemäss Anspruch 1, dadurch gekennzeichnet, dass Verbindungen der Formel I verwendet werden, worin A Wasserstoff und B Hydroxy bedeuten, X Halogen bedeutet, $R_2$ für Isovaleroyl oder β-Methylvaleroyl steht und von den Resten $R_1$ und $R_3$ einer für Acetyl und der andere für Isovaleroyl, β-Acetoxyisovaleroyl oder α-Isovaleroyloxyisovaleroyl steht.

13. Verwendung von Polyacyloxycyclopenta(c)-pyran-Verbindungen gemäss Anspruch 1 oder 2, dadurch gekennzeichnet, dass Verbindungen der Formel I verwendet werden, worin A und B gemeinsam eine Bindung bilden und $R_1$, $R_2$, $R_3$ und X die in Anspruch 1 oder 2 angegebenen Bedeutung besitzen.

14. Verwendung von Polyacyloxycyclopenta(c)-pyran-Verbindungen gemäss Anspruch 13, dadurch gekennzeichnet, dass Verbindungen der Formel I verwendet werden, worin A und B gemeinsam eine Bindung bilden, von den Resten $R_1$ und $R_3$ einer für Acetyl und der andere für Isovaleroyl oder β-Acetoxyisovaleroyl steht und $R_2$ Isovaleroyl oder β-Methylvaleroyl bedeutet und X die in Anspruch 13 angegebene Bedeutung besitzt.

15. Verfahren zur Herstellung von Arzneimitteln zur Behandlung und Prophylaxe von peptischen Ulcera und/oder zur Behandlung von krampfartigen Motilitätsstörungen im gastrointestinalen Trakt, dadurch gekennzeichnet, dass man eine ulcushemmend und/oder motilitätsnormalisierend wirksame Menge von Polyacyloxycyclopenta(c)-pyran-Verbindungen gemäss Anspruch 1 oder deren Gemischen zusammen mit üblichen pharmazeutischen Hilfsstoffen in eine geeignete Arzneiform überführt.

16. Polyacyloxycyclopenta(c)pyran-Verbindungen der allgemeinen Formel Ia

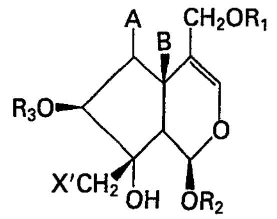

Ia

worin A für Wasserstoff und B für Wasserstoff oder Hydroxy stehen oder A und B gemeinsam eine Bindung bilden, von den Resten $R_1$ bis $R_3$ einer für Isovaleroyl, ein weiterer für Acetyl und der dritte für den Acylrest einer der folgenden Säuren steht: Isovaleriansäure, β-Methylvaleriansäure, α-Isovaleroyloxyisovaleriansäure, α-Acetoxyisovaleriansäure, β-Acetoxyisovaleriansäure, β-Acetoxy-β-methylvaleriansäure oder β-Hydroxyisovaleriansäure, und X' für Cyano, Azido oder einen Acyloxyrest $R_4COO$ steht, worin $R_4$ gegebenenfalls durch Hydroxy substituiertes Äthyl, Propyl, Isopropyl, n-Butyl, 1-Methylpropyl oder tert.-Butyl, eine gegebenenfalls durch Hydroxy substituierte Alkylgruppe mit 5 bis 20 Kohlenstoffatomen oder eine Alkenylgruppe mit 3 bis 20 Kohlenstoffatomen doer eine Phenyl-, Phenylalkyl- oder Phenylalkenylgruppe mit bis zu 3 Kohlenstoffatomen in der Alkylen- oder Alkenylenkette, welche gegebenenfalls im Phenylring durch Halogen, Trifluormethyl, Hydroxy, niederes Alkyl oder niederes Alkoxy substituiert sein kann, bedeutet, oder falls B Hydroxy darstellt, und/oder $R_2$ nicht Isovaleroyl ist und/oder wenn einer der Reste $R_1$ und $R_3$ Acetyl ist und der andere nicht Isovaleroyl ist, X' auch Halogen, Acetoxy oder Rhodano bedeuten kann, oder A und B gemeinsam eine Bindung bilden, X' Chlor bedeutet und $R_1$ und $R_2$ je Isovaleroyl und $R_3$ Acetyl oder $R_1$ Acetyl, $R_2$ Isovaleroyl und $R_3$ Acetoxyisovaleroyl bedeuten.

17. Polyacyloxycyclopenta(c)pyran-Verbindungen gemäss Anspruch 16, worin A, B und X' die in Anspruch 16 angegebene Bedeutung besitzen, $R_2$ Isovaleroyl oder β-Methylvaleroyl bedeutet, und von den Resten $R_1$ und $R_3$ einer für Acetyl und der andere für Isovaleroyl oder β-Acetoxyisovaleroyl oder, falls B = OH, auch einer für α-Isovaleroyloxyisovaleroyl und der andere für Acetyl steht.

18. Polyacyloxycyclopenta(c)pyran-Verbindungen gemäss Anspruch 17, worin X' eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

19. Polyacyloxycyclopenta(c)pyran-Verbindungen gemäss Anspruch 16, worin A und B je Wasserstoff bedeuten und $R_1$, $R_2$, $R_3$ und X' die in Anspruch 16 angegebene Bedeutung besitzen.

20. Polyacyloxycyclopenta(c)pyran-Verbindungen gemäss Anspruch 19, worin $R_1$, $R_2$, $R_3$, A und B die in Anspruch 19 angegebene Bedeutung besitzen und X' Halogen, Alkanoyloxy mit 2 bis 10 Kohlenstoffatomen oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

21. Polyacyloxycyclopenta(c)pyran-Verbindungen gemäss Anspruch 20, worin $R_1$ Isovaleroyl, $R_2$ Isovaleroyl oder β-Methylvaleroyl und $R_3$ Acetyl bedeuten und A, B und X die in Anspruch 20 angegebene Bedeutung besitzen.

22. Didrovaltrat- und/oder Homodidrovaltrathydrine gemäss Anspruch 21, worin X' Chlor oder Alkanoyloxy mit 2 bis 7 Kohlenstoffatomen bedeutet.

23. Didrovaltrat- und/oder Homodidrovaltrathydrine gemäss Anspruch 21, worin X' eine Benzoyloxy- oder Phenylacetoxygruppe bedeutet, deren Phenylring gegebenenfalls durch Chlor substituiert sein kann.

24. Verfahren zur Herstellung von Verbindungen der Formel Ia

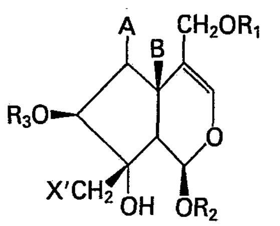

Ia

worin die Substituenten A, B, $R_1$–$R_3$ und X' die in Anspruch 16 genannte Bedeutung besitzen, dadurch gekennzeichnet, dass man Verbindungen der Formel II

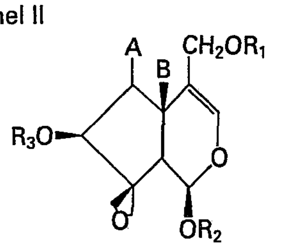

II

worin A, B, $R_1$, $R_2$ und $R_3$ die obige Bedeutung besitzen, mit einem Alkalimetallsalz oder einem quartären Ammoniumsalz einer Säure der Formel III

$$H{-}X' \qquad III$$

worin X' die obige Bedeutung besitzt, in einem unter den Reaktionsbedingungen inerten Lösungsmittel umsetzt.

25. Arzneimittel, dadurch gekennzeichnet, dass sie neben üblichen pharmazeutischen Hilfsstoffen als Wirkstoffe eine wirksame Menge von Polyacyloxycyclopenta(c)pyran-Verbindungen gemäss Anspruch 16 oder deren Gemischen enthalten.

26. Polyacyloxycyclopenta(c)pyran-Verbindungen der allgemeinen Formel Ib

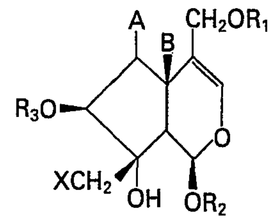

Ib

worin A für Wasserstoff und B für Wasserstoff oder Hydroxy stehen oder A und B gemeinsam eine Bindung bilden, von den Resten $R_1$ bis $R_3$ einer für Isovaleroyl, ein weiterer für Acetyl und der dritte für den Acylrest einer der folgenden Säuren steht: Isovaleriansäure, β-Methylvaleriansäure, α-Isovaleroyloxyisovaleriansäure, α-Acetoxyisovaleriansäure, β-Acetoxyisovaleriansäure, β-Acetoxy-β-methylvaleriansäure oder β-Hydroxyisovaleriansäure, und X für Halogen, Cyano, Rhodano, Azido oder einen Acyloxyrest $R_4COO$ steht, worin $R_4$ eine Alkylgruppe mit 1 bis 20 Kohlenstoffatomen, welche, falls A und B gemeinsam eine Bindung bilden, mindestens 6 Kohlenstoffatome enthält, eine gegebenenfalls durch Hydroxy substituierte Alkylgruppe mit 1 bis 20 Kohlenstoffatomen, eine Alkenylgruppe mit 3 bis 20 Kohlenstoffatomen oder eine Phenyl-, Phenylalkyl- oder Phenylalkenylgruppe mit bis zu 3 Kohlenstoffatomen in der Alkylen- oder Alkenylenkette, welche gegebenenfalls im Phenylring durch Halogen, Trifluormethyl, Hydroxy, niederes Alkyl oder niederes Alkoxy substituiert sein kann, bedeutet, zur Anwendung bei der Behandlung und Prophylaxe von peptischen Ulcera und/oder der Behandlung von krampfartigen Motilitätsstörungen im gastrointestinalen Trakt in grösseren Säugetieren und Menschen.

27. Polyacyloxycyclopenta(c)pyran-Verbindungen gemäss Anspruch 26, worin $R_1$, $R_2$, $R_3$, A und B die in Anspruch 26 angegebene Bedeutung besitzen und X Halogen, eine Alkanoyloxygruppe mit 2 bis 10 Kohlenstoffatomen, welche, falls A und B gemeinsam eine Bindung bilden, mindestens 7 Kohlenstoffatome enthält, oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

28. Polyacyloxycyclopenta(c)pyran-Verbindungen gemäss Anspruch 26 oder 27, worin A, B, $R_1$, $R_3$ und X die in Anspruch 26 oder 27 angegebene Bedeutung besitzen und $R_2$ Isovaleroyl oder β-Methylvaleroyl bedeutet.

29. Polyacyloxycyclopenta(c)pyran-Verbindungen gemäss Anspruch 28, worin A, B, X und $R_2$ die in Anspruch 28 angegebenen Bedeutung besitzen

und von den Resten $R_1$ und $R_3$ einer für Acetyl und der andere für Isovaleroyl oder β-Acetoxyisovaleroyl oder, falls B = OH, auch einer für α-Isovaleroyloxyisovaleroyl und der andere für Acetyl steht.

30. Polyacyloxycyclopenta(c)pyran-Verbindungen gemäss Anspruch 26 oder 27, worin A und B je Wasserstoff bedeuten und $R_1$, $R_2$, $R_3$ und X die in Anspruch 26 oder 27 angegebene Bedeutung besitzen.

31. Polyacyloxycyclopenta(c)pyran-Verbindungen gemäss Anspruch 26, worin A und B je Wasserstoff bedeuten, $R_1$ Isovaleroyl, $R_2$ Isovaleroyl oder β-Methylvaleroyl und $R_3$ Acetyl bedeuten und X die in Anspruch 26 angegebene Bedeutung besitzt.

32. Hydrine des Didrovaltratum und/oder des Homodidrovaltratum gemäss Anspruch 31, worin X Halogen, Alkanoyloxy mit 2 bis 10 Kohlenstoffatomen oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

33. Hydrine des Didrovaltratum und/oder des Homodidrovaltratum gemäss Anspruch 32, worin X eine Benzoyloxy- oder Phenylacetoxygruppe bedeutet, deren Phenylring gegebenenfalls durch Chlor substituiert sein kann.

34. Hydrine des Didrovaltratum und/oder des Homodidrovaltratum gemäss Anspruch 32, worin X Chlor bedeutet.

35. Hydrine des Didrovaltratum und/oder des Homodidrovaltratum gemäss Anspruch 32, worin X Alkanoyloxy mit 2 bis 7 Kohlenstoffatomen bedeutet.

36. Hydrine des Didrovaltratum und/oder des Homodidrovaltratum gemäss Anspruch 32, worin X Isovaleroyloxy bedeutet.

37. Polyacyloxycyclopenta(c)pyran-Verbindungen gemäss Anspruch 26, worin A Wasserstoff und B Hydroxy bedeuten, X Halogen bedeutet, $R_2$ für Isovaleroyl oder β-Methylvaleroyl steht und von den Resten $R_1$ und $R_3$ einer für Acetyl und der andere für Isovaleroyl, β-Acetoxyisovaleroyl oder α-Isovaleroyloxyisovaleroyl steht.

38. Polyacyloxycyclopenta(c)pyran-Verbindungen gemäss Anspruch 26 oder 27, worin A und B gemeinsam eine Bindung bilden und $R_1$, $R_2$, $R_3$ und X die in Anspruch 26 oder 27 angegebene Bedeutung besitzen.

39. Polyacyloxycyclopenta(c)pyran-Verbindungen gemäss Anspruch 38, worin A und B gemeinsam eine Bindung bilden, von den Resten $R_1$ und $R_3$ einer für Acetyl und der andere für Isovaleroyl oder β-Acetoxyisovaleroyl steht und $R_2$ Isovaleroyl oder β-Methylvaleroyl bedeutet und X die in Anspruch 38 angegebene Bedeutung besitzt.

**Patentansprüche für den Vertragsstaat AT**

1. Verwendung von Polyacyloxycyclopenta(c)-pyran-Verbindungen der allgemeinen Formel I

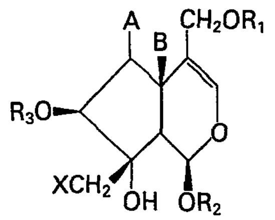

I

worin A für Wasserstoff und B für Wasserstoff oder Hydroxy stehen oder A und B gemeinsam eine Bindung bilden, von den Resten $R_1$ bis $R_3$ einer für Isovaleroyl, ein weiterer für Acetyl und der dritte für den Acylrest einer der folgenden Säuren steht: Isovaleriansäure, β-Methylvaleriansäure, α-Isovaleroyloxyisovaleriansäure, α-Acetoxyisovaleriansäure, β-Acetoxyisovaleriansäure, β-Acetoxy-β-methylvaleriansäure oder β-Hydroxyisovaleriansäure, und X für Halogen, Cyano, Rhodano, Azido oder einen Acyloxyrest $R_4COO$ steht, worin $R_4$ eine gegebenenfalls durch Hydroxy substituierte Alkylgruppe mit 1 bis 20 Kohlenstoffatomen oder eine Alkenylgruppe mit 3 bis 20 Kohlenstoffatomen oder eine Phenyl-, Phenylalkyl- oder Phenylalkenylgruppe mit bis zu 3 Kohlenstoffatomen in der Alkylen- oder Alkenylenkette welche gegebenenfalls im Phenylring durch Halogen, Trifluormethyl, Hydroxy, niederes Alkyl oder niederes Alkoxy substituiert sein kann, bedeutet, zur Herstellung von pharmazeutischen Zubereitungen zur Behandlung und Prophylaxe von peptischen Ulcera und/oder zur Behandlung von krampfartigen Motilitätsstörungen im gastrointestinalen Trakt in grösseren Säugetieren und Menschen.

2. Verwendung von Polyacyloxycyclopenta(c)-pyran-Verbindungen gemäss Anspruch 1, dadurch gekennzeichnet, dass Polyacyloxycyclopenta(c)pyran-Verbindungen der Formel I verwendet werden, worin $R_1$, $R_2$, $R_3$, A und B die in Anspruch 1 angegebene Bedeutung besitzen und X Halogen, Alkanoyloxy mit 2 bis 10 Kohlenstoffatomen oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

3. Verwendung von Polyacyloxycyclopenta(c)-pyran-Verbindungen gemäss Anspruch 1 oder 2, dadurch gekennzeichnet, dass Polyacyloxycyclopenta(c)pyran-Verbindungen der Formel I verwendet werden, worin A, B, $R_1$, $R_3$ und X die in Anspruch 1 oder 2 angegebenen Bedeutung besitzen und $R_2$ Isovaleroyl oder β-Methylvaleroyl bedeutet.

4. Verwendung von Polyacyloxycyclopenta(c)-pyran-Verbindungen gemäss Anspruch 3, dadurch gekennzeichnet, dass Polyacyloxycyclopenta(c)pyran-Verbindungen verwendet werden, worin A, B, X und $R_2$ die in Anspruch 3 angegebenen Bedeutung besitzen und von den Resten $R_1$ und $R_3$ einer für Acetyl und der andere für Isovaleroyl oder β-Acetoxyisovaleroyl oder, falls B = OH, auch einer für α-Isovaleroyloxyisovaleroyl und der andere für Acetyl steht.

5. Verwendung von Polyacyloxycyclopenta(c)-pyran-Verbindungen gemäss Anspruch 1 oder 2, dadurch gekennzeichnet, dass Polyacyloxycyclopenta(c)pyran-Verbindungen der Formel I verwendet werden, worin A und B je Wasserstoff bedeuten und $R_1$, $R_2$, $R_3$ und X die in Anspruch 1 oder 2 angegebene Bedeutung besitzen.

6. Verwendung von Polyacyloxycyclopenta(c)-pyran-Verbindungen gemäss Anspruch 1, dadurch gekennzeichnet, dass Polyacyloxycyclopenta(c)pyran-Verbindungen der Formel I verwendet werden, worin A und B je Wasserstoff bedeuten, $R_1$ Isovaleroyl, $R_2$ Isovaleroyl oder β-Methylvaleroyl und $R_3$ Acetyl bedeuten und X die in Anspruch 1 angegebene Bedeutung besitzt.

7. Verwendung von Polyacyloxycyclopenta(c)-pyran-Verbindungen gemäss Anspruch 6, dadurch gekennzeichnet, dass Hydrine des Didrovaltratum und/oder des Homodidrovaltratum verwendet werden, worin X Halogen, Alkanoyloxy mit 2 bis 10 Kohlenstoffatomen oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

8. Verwendung von Polyacyloxycyclopenta(c)-pyran-Verbindungen gemäss Anspruch 7, dadurch gekennzeichnet, dass Didrovaltratchlorhydrin und/oder Homodidrovaltratchlorhydrin verwendet werden.

9. Verwendung von Polyacyloxycyclopenta(c)-pyran-Verbindungen gemäss Anspruch 7, dadurch gekennzeichnet, dass Didrovaltratalkanoyloxyhydrine und/oder Homodidrovaltratalkanoyloxyhydrine mit einem Alkanoyloxyrest mit 2 bis 7 Kohlenstoffatomen verwendet werden.

10. Polyacyloxycyclopenta(c)pyran-Verbindungen gemäss Anspruch 9, worin der Alkanoyloxyrest Isovaleroyloxy darstellt.

11. Verwendung von Polyacyloxycyclopenta(c)-pyran-Verbindungen gemäss Anspruch 7, dadurch gekennzeichnet, dass Hydrine des Didrovaltratum und/oder des Homodidrovaltratum verwendet werden, worin X eine Benzoyloxy- oder Phenylacetoxygruppe bedeutet, deren Phenylring gegebenenfalls durch Chlor substituiert sein kann.

12. Verwendung von Polyacyloxycyclopenta(c)-pyran-Verbindungen gemäss Anspruch 1, dadurch gekennzeichnet, dass Verbindungen der Formel I verwendet werden, worin A Wasserstoff und B Hydroxy bedeuten, X Halogen bedeutet, $R_2$ für Isovaleroyl oder β-Methylvaleroyl steht und von den Resten $R_1$ und $R_3$ einer für Acetyl und der andere für Isovaleroyl, β-Acetoxyisovaleroyl oder α-Isovaleroyloxyisovaleroyl steht.

13. Verwendung von Polyacyloxycyclopenta(c)-pyran-Verbindungen gemäss Anspruch 1 oder 2, dadurch gekennzeichnet, dass Verbindungen der Formel I verwendet werden, worin A und B gemeinsam eine Bindung bilden und $R_1$, $R_2$, $R_3$ und X die in Anspruch 1 oder 2 angegebenen Bedeutung besitzen.

14. Verwendung von Polyacyloxycyclopenta(c)-pyran-Verbindungen gemäss Anspruch 13, dadurch gekennzeichnet, dass Verbindungen der Formel I verwendet werden, worin A und B gemeinsam eine Bindung bilden, von den Resten $R_1$ und $R_3$ einer für Acetyl und der andere für Isovaleroyl oder β-Acetoxyisovaleroyl steht und $R_2$ Isovaleroyl oder β-Methylvaleroyl bedeutet, und X die in Anspruch 13 angegebene Bedeutung besitzt.

15. Verfahren zur Herstellung von Arzneimitteln zur Behandlung und Prophylaxe von peptischen Ulcera und/oder zur Behandlung von krampfartigen Motilitätsstörungen im gastrointestinalen Trakt, dadurch gekennzeichnet, dass man eine ulcushemmend und/oder motilitätsnormalisierend wirksame Menge von Polyacyloxycyclopenta(c)-pyran-Verbindungen gemäss Anspruch 1 oder deren Gemischen zusammen mit üblichen pharmazeutischen Hilfsstoffen in eine geeignete Arzneiform überführt.

16. Verfahren zur Herstellung von Polyacyloxycyclopenta(c)pyran-Verbindungen der allgemeinen Formel Ia

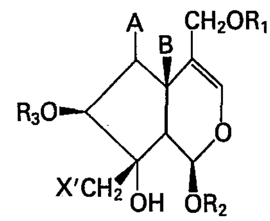

Ia

worin A für Wasserstoff und B für Wasserstoff oder Hydroxy stehen oder A und B gemeinsam eine Bindung bilden, von den Resten $R_1$ bis $R_3$ einer für Isovaleroyl, ein weiterer für Acetyl und der dritte für den Acylrest eine der folgenden Säuren steht: Isovaleriansäure, β-Methylvaleriansäure, α-Isovaleroyloxyisovaleriansäure, α-Acetoxyisovaleriansäure, β-Acetoxyisovaleriansäure, β-Acetoxy-β-methylvaleriansäure oder β-Hydroxyisovaleriansäure, und X' für Cyano, Azido oder einen Acyloxyrest $R_4COO$ steht, worin $R_4$ gegebenenfalls durch Hydroxy substituiertes Äthyl, Propyl, Isopropyl, n-Butyl, 1-Methylpropyl oder tert.-Butyl, eine gegebenenfalls durch Hydroxy substituierte Alkylgruppe mit 5 bis 20 Kohlenstoffatomen oder eine Alkenylgruppe mit 3 bis 20 Kohlenstoffatomen oder eine Phenyl-, Phenylalkyl- oder Phenylalkenylgruppe mit bis zu 3 Kohlenstoffatomen in der Alkylen- oder Alkenylenkette, welche gegebenenfalls im Phenylring durch Halogen, Trifluormethyl, Hydroxy, niederes Alkyl oder niederes Alkoxy substituiert sein kann, bedeutet, oder falls B Hydroxy darstellt, und/oder $R_2$ nicht Isovaleroyl ist und/oder wenn einer der Reste $R_1$ und $R_3$ Acetyl ist und der andere nicht Isovaleroyl ist, X' auch Halogen, Acetoxy oder Rhodano bedeuten kann, oder A und B gemeinsam eine Bindung bilden, X' Chlor bedeutet und $R_1$ und $R_2$ je Isovaleroyl und $R_3$ Acetyl oder $R_1$ Acetyl, $R_2$ Isovaleroyl und $R_3$ Acetoxyisovaleroyl bedeuten, dadurch gekennzeichnet, dass man Verbindungen der Formel II

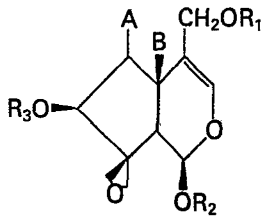

II

worin A, B, $R_1$, $R_2$ und $R_3$ obige Bedeutung besitzen, mit einem Alkalimetallsalz oder einem quartären Ammoniumsalz einer Säure der Formel III

H–X' III

worin X' obige Bedeutung besitzt, in einem unter den Reaktionsbedingungen inerten Lösungsmittel umsetzt.

17. Verfahren nach Anspruch 16, dadurch gekennzeichnet, dass Polyacyloxycyclopenta(c)pyran-Verbindungen gemäss Anspruch 16 hergestellt werden, worin A, B und X' die in Anspruch 16 angegebene Bedeutung besitzen, $R_2$ Isovaleroyl oder β-Methylvaleroyl bedeutet, und von den Resten $R_1$ und $R_3$ einer für Acetyl und der andere für Isovaleroyl oder β-Acetoxyisovaleroyl oder, falls B = OH, auch einer für α-Isovaleroyloxyisovaleroyl und der andere für Acetyl steht.

18. Verfahren nach Anspruch 16, dadurch gekennzeichnet, dass Polyacyloxycyclopenta(c)pyran-Verbindungen gemäss Anspruch 17 hergestellt werden, worin X' eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

19. Verfahren nach Anspruch 16, dadurch gekennzeichnet, dass Polyacyloxycyclopenta(c)pyran-Verbindungen gemäss Anspruch 16 hergestellt werden, worin A und B je Wasserstoff bedeuten und $R_1$, $R_2$, $R_3$ und X' die in Anspruch 16 angegebene Bedeutung besitzen.

20. Verfahren nach Anspruch 16, dadurch gekennzeichnet, dass Polyacyloxycyclopenta(c)pyran-Verbindungen gemäss Anspruch 19 hergestellt werden, worin $R_1$, $R_2$, $R_3$, A und B die in Anspruch 19 angegebene Bedeutung besitzen und X' Halogen, Alkanoyloxy mit 2 bis 10 Kohlenstoffatomen oder eine Benzoyloxy-, Phenylalkanoyloxy- oder Phenylalkenoyloxygruppe mit bis zu 3 Kohlenstoffatomen in dem Alkanoyloxy- oder Alkenoyloxyrest, welche gegebenenfalls im Phenylring durch Halogen, niederes Alkoxy oder niederes Alkyl substituiert sein kann, bedeutet.

21. Verfahren nach Anspruch 20, dadurch gekennzeichnet, dass Polyacyloxycyclopenta(c)pyran-Verbindungen gemäss Anspruch 20 hergestellt werden, worin $R_1$ Isovaleroyl, $R_2$ Isovaleroyl oder β-Methylvaleroyl und $R_3$ Acetyl bedeuten und A, B und X die in Anspruch 20 angegebene Bedeutung besitzen.

22. Verfahren nach Anspruch 21, dadurch gekennzeichnet, dass als Verbindungen der Formel Ia Didrovaltrat- und/oder Homodidrovaltrathydrine gemäss Anspruch 21 hergestellt werden, worin X' Chlor oder Alkanoyloxy mit 2 bis 7 Kohlenstoffatomen bedeutet.

23. Verfahren nach Anspruch 21, dadurch gekennzeichnet, dass als Verbindungen der Formel Ia Didrovaltrat- und/oder Homodidrovaltrathydrine gemäss Anspruch 21 hergestellt werden, worin X' eine Benzoyloxy- oder Phenylacetoxygruppe bedeutet, deren Phenylring gegebenenfalls durch Chlor substituiert sein kann.

**Claims for the contracting States BE, CH, DE, FR, GB, IT, LI, NL**

1. Use of polyacyloxycyclopenta(c)pyran compounds of the general formula I

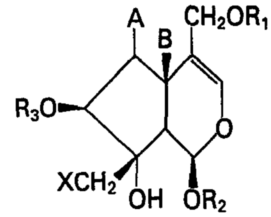

I

in which A stands for hydrogen and B stands for hydrogen or hydroxy or A and B together form a bond, of the radicals $R_1$ to $R_3$ one stands for isovaleroyl, another stands for acetyl and the third stands for the acyl radical of one of the following acids: isovaleric acid, β-methylvaleric acid, α-isovaleroyloxyisovaleric acid, α-acetoxyisovaleric acid, β-acetoxyisovaleric acid, β-acetoxy-β-methylvaleric acid or β-hydroxyisovaleric acid, and X stands for halogen, cyano, thiocyano, azido or an acyloxy radical $R_4COO$, in which $R_4$ is an alkyl group, optionally substituted with hydroxy, with 1 to 20 carbon atoms or an alkenyl group with 3–20 carbon atoms or a phenyl, phenylalkyl or phenylalkenyl group with up to 3 carbon atoms in the alkylene or alkenylene chain, which may be optionally substituted in the phenyl ring with halogen, trifluoromethyl, hydroxy, lower alkyl or lower alkoxy, for the production of pharmaceutical preparations for the treatment and prophylaxis of peptic ulcers and/or for the treatment of spasmodic motility disorders in the gastrointestinal tract in larger mammals and humans.

2. Use of polyacyloxycyclopenta(c)pyran compounds according to claim 1, characterized in that polyacyloxycyclopenta(c)pyran compounds of the formula I are used, in which $R_1$, $R_2$, $R_3$, A and B have the meaning given in claim 1 and X is halogen, alkanoyloxy with 2 to 10 carbon atoms or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

3. Use of polyacyloxycyclopenta(c)pyran compounds according to claim 1 or 2, characterized in that polyacyloxycyclopenta(c)pyran compounds of the formula I are used, in which A, B, $R_1$, $R_3$ and X have the meaning given in claim 1 or 2, and $R_2$ is isovaleroyl or β-methylvaleroyl.

4. Use of polyacyloxycyclopenta(c)pyran compounds according to claim 3, characterized in that polyacyloxycyclopenta(c)pyran compounds are used in which A, B, X and $R_2$ have the meaning given in claim 3 and of the radicals $R_1$ and $R_3$ one stands for acetyl and the other stands for isovaleroyl or β-acetoxyisovaleroyl or, if B = OH, also one stands for α-isovaleroyloxyisovaleroyl and the other for acetyl.

5. Use of polyacyloxycyclopenta(c)pyran compounds according to claim 1 or 2, characterized in that polyacyloxycyclopenta(c)pyran compounds of formula I are used, in which A and B are each hydrogen and $R_1$, $R_2$, $R_3$ and X have the meaning given in claim 1 or 2.

6. Use of polyacyloxycyclopenta(c)pyran compounds according to claim 1, characterized in that polyacyloxycyclopenta(c)pyran compounds of formula I are used, in which A and B are each hydrogen, $R_1$ is isovaleroyl, $R_2$ is isovaleroyl or β-methylvaleroyl and $R_3$ is acetyl and X has the meaning given in claim 1.

7. Use of polyacyloxycyclopenta(c)pyran compounds according to claim 6, characterized in that hydrins of didrovaltratum and/or of homodidrovaltratum are used, in which X is halogen, alkanoyloxy with 2 to 10 carbon atoms or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

8. Use of polyacyloxycyclopenta(c)pyran compounds according to claim 7, characterized in that didrovaltratochlorohydrin and/or homodidrovaltratochlorohydrin are used.

9. Use of polyacyloxycyclopenta(c)pyran compounds according to claim 7, characterized in that didrovaltratoalkanoyloxyhydrins and/or homodidrovaltratoalkanoyloxyhydrins with an alkanoyloxy radical with 2 to 7 carbon atoms are used.

10. Use of polyacyloxycyclopenta(c)pyran compounds according to claim 9, in which the alkanoyloxy radical represents isovayleroyloxy.

11. Use of polyacyloxycyclopenta(c)pyran compounds according to claim 7, characterized in that hydrins of didrovaltratum and/or of homodidrovaltratum are used, in which X is a benzoyloxy or phenylacetoxy group, the phenyl ring of which may be optionally substituted with chlorine.

12. Use of polyacyloxycyclopenta(c)pyran compounds according to claim 1, characterized in that compounds of formula I are used, in which A is hydrogen and B is hydroxy, X is halogen, $R_2$ stands for isovaleroyl or β-methylvaleroyl and of the radicals $R_1$ and $R_3$ one stands for acetyl and the other stands for isovaleroyl, β-acetoxyisovaleroyl or α-isovaleroyloxyisovaleroyl.

13. Use of polyacyloxycyclopenta(c)pyran compounds according to claim 1 or 2, characterized in that compounds of formula I are used, in which A and B together form a bond and $R_1$, $R_2$, $R_3$ and X have the meaning given in claim 1 or 2.

14. Use of polyacyloxycyclopenta(c)pyran compounds according to claim 13, characterized in that compounds of formula I are used, in which A and B together form a bond, of the radicals $R_1$ and $R_3$ one stands for acetyl and the other for isovaleroyl or β-acetoxyisovaleroyl and $R_2$ is isovaleroyl or β-methylvaleroyl and X has the meaning given in claim 13.

15. Method for the production of medicaments for the treatment and prophylaxis of peptic ulcers and/or for the treatment of spasmodic motility disorders in the gastrointestinal tract, characterized in that an ulcer-inhibiting and/or motility-normalising effective quantity of polyacyloxycyclopenta(c)pyran compounds according to claim 1 or mixtures thereof together with conventional pharmaceutical adjuvants is converted into a suitable medical form.

16. Polyacyloxycyclopenta(c)pyran compounds of the general formula Ia

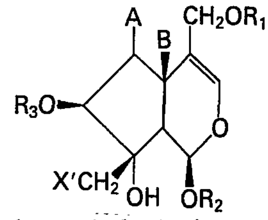

Ia

in which A stands for hydrogen and B stands for hydrogen or hydroxy or A and B together form a bond, of the radicals $R_1$ and $R_3$ one stands for isovaleroyl, another stands for acetyl and the third stands for the acyl radical of one of the following acids: Isovaleric acid, β-methylvaleric acid, α-isovaleroyloxyisovaleric acid, α-acetoxyisovaleric acid, β-acetoxyisovaleric acid, β-acetoxy-β-methylvaleric acid or β-hydroxyisovaleric acid, and X' stands for cyano, azido or an acyloxy radical $R_4COO$, in which $R_4$ is an ethyl, propyl, isopropyl, n-butyl, 1-methylpropyl or tert.-butyl group which is optionally substituted with hydroxy, an alkyl group with 5 to 20 carbon atoms optionally substituted with hydroxy, or an alkenyl group with 3 to 20 carbon atoms or a phenyl, phenylalkyl or phenylalkenyl group with up to 3 carbon atoms in the alkylene or alkenylene chain, which may be optionally substituted in the phenyl ring with halogen, trifluoromethyl, hydroxy, lower alkyl or lower alkoxy, or if B represents hydroxy, and/or $R_2$ is not isovaleroyl and/or if one of the radicals $R_1$ and $R_3$ is acetyl and the other is not isovaleroyl, X' may also be halogen, acetoxy or thiocyano, or A and B together form a bond, X' is chlorine and $R_1$ and $R_2$ are each isovaleroyl and $R_3$ is acetyl or $R_1$ is acetyl, $R_2$ is isovaleroyl and $R_3$ is acetoxyisovaleroyl.

17. Polyacyloxycyclopenta(c)pyran compounds according to claim 16, in which A, B and X' have the meaning given in claim 16, $R_2$ is isovaleroyl or β-methylvaleroyl, and of the radicals $R_1$ and $R_3$ one stands for acetyl and the other stands for isovaleroyl or β-acetoxyisovaleroyl or, if B = OH, also one stands for α-isovaleroyloxyisovaleroyl and the other stands for acetyl.

18. Polyacyloxycyclopenta(c)pyran compounds according to claim 17, in which X' is a benzoyloxy, phenylalkanoyloxy or phenylalkenoyloxy group with up to 3 carbon atoms in the alkanoyloxy or

alkenoyloxy radical, which may be optionally substituted in the phenyl ring with halogen, lower alkoxy or lower alkyl.

19. Polyacyloxycyclopenta(c)pyran compounds according to claim 16, in which A and B are each hydrogen and $R_1$, $R_2$, $R_3$ and X' have the meaning given in claim 16.

20. Polyacyloxycyclopenta(c)pyran compounds according to claim 19, in which $R_1$, $R_2$, $R_3$, A and B have the meaning given in claim 19 and X' is halogen, alkanoyloxy with 2 to 10 carbon atoms or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

21. Polyacyloxycyclopenta(c)pyran compounds according to claim 20, in which $R_1$ is isovaleroyl, $R_2$ denotes isovaleroyl or β-methylvaleroyl and $R_3$ is acetyl and A, B and X have the meaning given in claim 20.

22. Didrovaltrato- and/or homodidrovaltratohydrins according to claim 21, in which X' is chlorine or alkanoyloxy with 2 to 7 carbon atoms.

23. Didrovaltrato- and/or homodidrovaltratohydrins according to claim 21, in which X' is a benzoyloxy or phenylacetoxy group, the phenyl ring of which may be optionally substituted with chlorine.

24. Method for the production of compounds of formula Ia

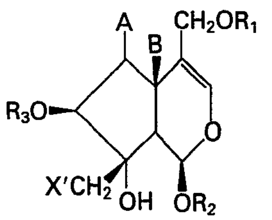

Ia

in which the substituents A, B, $R_1$–$R_3$ and X' have the meaning given in claim 16, characterized in that compounds of formula II

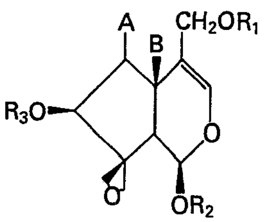

II

in which A, B, $R_1$, $R_2$ and $R_3$ have the above meaning, are reacted with an alkali metal salt or with a quaternary ammonium salt of an acid of formula III

H–X' III

in which X' has the above meaning, in a solvent which is inert under the reaction conditions.

25. Medicaments characterized in that in addition to conventional pharmaceutical adjuvants, they contain as effective substances an effective quantity of polyacyloxycyclopenta(c)pyran compounds according to claim 16 or mixtures thereof.

26. Polyacyloxycyclopenta(c)pyran compounds of the general formula Ib

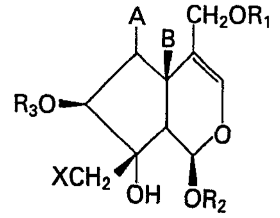

Ib

in which A stands for hydrogen and B stands for hydrogen or hydroxy or A and B together form a bond, of the radicals $R_1$ to $R_3$ one stands for isovaleroyl, another stands for acetyl and the third stands for the acyl radical of one of the following acids: isovaleric acid, β-methylvaleric acid, α-isovaleroyloxyisovaleric acid, α-acetoxyisovaleric acid, β-acetoxyisovaleric acid, β-acetoxy-β-methylvaleric acid or β-hydroxyisovaleric acid, and X stands for halogen, cyano, thiocyano, azido or an acyloxy radical $R_4COO$, in which $R_4$ is an alkyl group with 1 to 20 carbon atoms which, if A and B together form a bond, contains at least 6 carbon atoms, an alkyl group with 1 to 20 carbon atoms optionally substituted with hydroxy, an alkenyl group with 3 to 20 carbon atoms or a phenyl, phenylalkyl or phenylalkenyl group with up to 3 carbon atoms in the alkylene or alkenylene chain, which may be optionally substituted in the phenyl ring with halogen, trifluoromethyl, hydroxy, lower alkyl or lower alkoxy, for use in the treatment and prophylaxis of peptic ulcers and/or for the treatment of spasmodic motility disorders in the gastrointestinal tract in larger mammals and humans.

27. Polyacyloxycyclopenta(c)pyran compounds according to claim 26, in which $R_1$, $R_2$, $R_3$, A and B have the meaning given in claim 26 and X is halogen, an alkanoyloxy group with 2 to 10 carbon atoms, which, if A and B together form a bond, contains at least 7 carbon atoms, or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

28. Polyacyloxycyclopenta(c)pyran compounds according to claim 26 or 27, in which A, B, $R_1$, $R_3$ and X have the meaning given in claim 26 or 27, and $R_2$ is isovaleroyl or β-methylvaleroyl.

29. Polyacyloxycyclopenta(c)pyran compounds according to claim 28, in which A, B, X and $R_2$ have the meaning given in claim 28 and of the radicals $R_1$ and $R_3$ one stands for acetyl and the other stands for isovaleroyl or β-acetoxyisovaleroyl or, if B = OH, also one stands for α-isovaleroyloxy-isovaleroyl and the other for acetyl.

30. Polyacyloxycyclopenta(c)pyran compounds according to claim 26 or 27, in which A and B are each hydrogen and $R_1$, $R_2$, $R_3$ and X have the meaning given in claim 26 or 27.

31. Polyacyloxycyclopenta(c)pyran compounds according to claim 26, in which A and B are each hydrogen, $R_1$ is isovaleroyl, $R_2$ is isovaleroyl or β-methylvaleroyl and $R_3$ is acetyl and X has the meaning given in claim 26.

32. Hydrines of didrovaltratum and/or homodidrovaltratum according to claim 31, in which X is halogen, alkanoyloxy with 2 to 10 carbon atoms or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

33. Hydrines of didrovaltratum and/or homodidrovaltratum according to claim 32, in which X is a benzoyloxy or phenylacetoxy group, the phenyl ring of which may be optionally substituted with chlorine.

34. Hydrines of didrovaltratum and/or homodidrovaltratum according to claim 32, in which X is chlorine.

35. Hydrines of didrovaltratum and/or homodidrovaltratum according to claim 32, in which X is alkanoyloxy with 2 to 7 carbon atoms.

36. Hydrines of didrovaltratum and/or homodidrovaltratum according to claim 32, in which X is isovaleroyloxy.

37. Use of polyacyloxycyclopenta(c)pyran compounds according to claim 26, in which A is hydrogen an B is hydroxy, X is halogen, $R_2$ stands for isovaleroyl or β-methylvaleroyl and of the radicals $R_1$ and $R_3$ one stands for acetyl and the other stands for isovaleroyl, β-acetoxyisovaleroyl or α-isovaleroyloxyisovaleroyl.

38. Polyacyloxycyclopenta(c)pyran compounds according to claim 26 or 27, in which A and B together form a bond and $R_1$, $R_2$, $R_3$ and X have the meaning given in claim 26 or 27.

39. Polyacyloxycyclopenta(c)pyran compounds according to claim 38, in which A and B together form a bond, of the radicals $R_1$ and $R_3$, one stands for acetyl and the other stands for isovaleroyl or β-acetoxyisovaleroyl and $R_2$ is isovaleroyl or β-methylvaleroyl and X has the meaning given in claim 38.

**Claims for the contracting State: AT**

1. Use of polyacyloxycyclopenta(c)pyran compounds of the general formula I

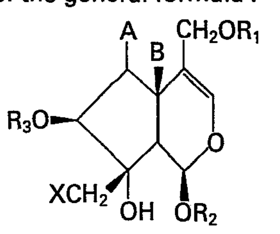

I

in which A stands for hydrogen and B stands for hydrogen or hydroxy or A and B together form a bond, of the radicals $R_1$ to $R_3$ one stands for isovaleroyl, another stands for acetyl and the third stands for the acyl radical of one of the following acids: isovaleric acid, β-methylvaleric acid, α-isovaleroyloxyisovaleric acid, α-acetoxyisovaleric acid, β-acetoxyisovaleric acid, β-acetoxy-β-methylvaleric acid or β-hydroxyisovaleric acid, and X stands for halogen, cyano, thiocyano, azido or an acyloxy radical $R_4COO$, in which $R_4$ is an alkyl group, optionally substituted with hydroxy, with 1 to 20 carbon atoms or an alkenyl group with 3–20 carbon atoms or a phenyl, phenylalkyl or phenylalkenyl group with up to 3 carbon atoms in the alkylene or alkenylene chain, which may be optionally substituted in the phenyl ring with halogen, trifluoromethyl, hydroxy, lower alkyl or lower alkoxy, for the production of pharmaceutical preparations for the treatment and prophylaxis of peptic ulcers and/or for the treatment of spasmodic motility disorders in the gastrointestinal tract in larger mammals and humans.

2. Use of polyacyloxycyclopenta(c)pyran compounds according to claim 1, characterized in that polyacyloxycyclopenta(c)pyran compounds of the formula I are used, in which $R_1$, $R_2$, $R_3$, A and B have the meaning given in claim 1 and X is halogen, alkanoyloxy with 2 to 10 carbon atoms or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

3. Use of polyacyloxycyclopenta(c)pyran compounds according to claim 1 or 2, characterized in that polyacyloxycyclopenta(c)pyran compounds of the formula I are used, in which A, B, $R_1$, $R_3$ and X have the meaning given in claim 1 or 2, and $R_2$ is isovaleroyl or β-methylvaleroyl.

4. Use of polyacyloxycyclopenta(c)pyran compounds according to claim 3, characterized in that polyacyloxycyclopenta(c)pyran compounds are used in which A, B, X and $R_2$ have the meaning given in claim 3 and of the radicals $R_1$ and $R_3$ one stands for acetyl and the other stands for isovaleroyl or β-acetoxyisovaleroyl or, if B = OH, also one stands for α-isovaleroyloxyisovaleroyl and the other for acetyl.

5. Use of polyacyloxycyclopenta(c)pyran compounds according to claim 1 or 2, characterized in that polyacyloxycyclopenta(c)pyran compounds of formula I are used, in which A and B are each hydrogen and $R_1$, $R_2$, $R_3$ and X have the meaning given in claim 1 or 2.

6. Use of polyacyloxycyclopenta(c)pyran compounds according to claim 1, characterized in that polyacyloxycyclopenta(c)pyran compounds of formula I are used, in which A and B are each hydrogen, $R_1$ is isovaleroyl, $R_2$ is isovaleroyl or β-methylvaleroyl and $R_3$ is acetyl and X has the meaning given in claim 1.

7. Use of polyacyloxycyclopenta(c)pyran compounds according to claim 6, characterized in that hydrins of didrovaltratum and/or of homodidrovaltratum are used, in which X is halogen, alkanoyloxy with 2 to 10 carbon atoms or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

8. Use of polyacyloxycyclopenta(c)pyran compounds according to claim 7, characterized in that didrovaltratochlorohydrin, and/or homodidrovaltratochlorohydrin are used.

9. Use of polyacyloxycyclopenta(c)pyran compounds according to claim 7, characterized in that didrovaltratoalkanoyloxyhydrins and/or homodidrovaltratoalkanoyloxyhydrins with an alkanoyloxy radical with 2 to 7 carbon atoms are used.

10. Use of polyacyloxycyclopenta(c)pyran compounds according to claim 9, in which the alkanoyloxy radical represents isovaleroyloxy.

11. Use of polyacyloxycyclopenta(c)pyran compounds according to claim 7, characterized in that hydrins of didrovaltratum and/or of homodidrovaltratum are used, in which X is a benzoyloxy or phenylacetoxy group, the phenyl ring of which may be optionally substituted with chlorine.

12. Use of polyacyloxycyclopenta(c)pyran compounds according to claim 1, characterized in that compounds of formula I are used, in which A is hydrogen and B is hydroxy, X is halogen, $R_2$ stands for isovaleroyl or β-methylvaleroyl and of the radicals $R_1$ and $R_3$ one stands for acetyl and the other stands for isovaleroyl, β-acetoxyisovaleroyl or α-isovaleroyloxyisovaleroyl.

13. Use of polyacyloxycyclopenta(c)pyran compounds according to claim 1 or 2, characterized in that compounds of formula I are used, in which A and B together form a bond and $R_1$, $R_2$, $R_3$ and X have the meaning given in claim 1 or 2.

14. Use of polyacyloxycyclopenta(c)pyran compounds according to claim 13, characterized in that compounds of formula I are used, in which A and B together form a bond, of the radicals $R_1$ and $R_3$ one stands for acetyl and the other for isovaleroyl or -acetoxyisovaleroyl and $R_2$ is isovaleroyl or β-methylvaleroyl and X has the meaning given in claim 13.

15. Method for the production of medicaments for the treatment and prophylaxis of peptic ulcers and/or for the treatment of spasmodic motility disorders in the gastrointestinal tract, characterized in that an ulcer-inhibiting and/or motility-normalising effective quantity of polyacyloxycyclopenta(c)pyran compounds according to claim 1 or mixtures thereof together with conventional pharmaceutical adjuvants is converted into a suitable medical form.

16. Method for the production of polyacyloxycyclopenta(c)pyran compounds of the general formula Ia

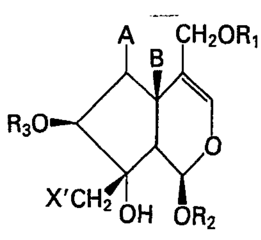

Ia

in which A stands for hydrogen and B stands for hydrogen or hydroxy or A and B together form a bond, of the radicals $R_1$ and $R_3$ one stands for isovaleroyl, another stands for acetyl and the third stands for the acyl radical of one of the following acids: isovaleric acid, β-methylvaleric acid, α-isovaleroyloxyisovaleric acid, α-acetoxyisovaleric acid, β-acetoxyisovaleric acid, β-acetoxy-β-methylvaleric acid or β-hydroxyisovaleric acid, and X' stands for cyano, azido or an acyloxy radical $R_4COO$, in which $R_4$ is an ethyl, propyl, isopropyl, n-butyl, 1-methylpropyl or tert.-butyl group which is optionally substituted with hydroxy, an alkyl group with 5 to 20 carbon atoms optionally substituted with hydroxy, or an alkenyl group with 3 to 20 carbon atoms or a phenyl, phenylalkyl or phenylalkenyl group with up to 3 carbon atoms in the alkylene or alkenylene chain, which may be optionally substituted in the phenyl ring with halogen, trifluoromethyl, hydroxy, lower alkyl or lower alkoxy, or if B represents hydroxy, and/or $R_2$ is not isovaleroyl and/or if one of the radicals $R_1$ and $R_3$ is acetyl and the other is not isovaleroyl, X' may also be halogen, acetoxy or thiocyano, or A and B together form a bond, X' is chlorine and $R_1$ and $R_2$ are each isovaleroyl and $R_3$ is acetyl or $R_1$ is acetyl, $R_2$ is isovaleroyl and $R_3$ is acetoxyisovaleroyl, characterized in that compounds of formula II

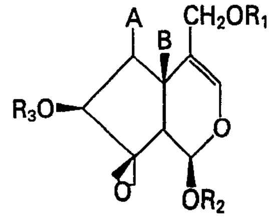

II

in which A, B, $R_1$, $R_2$ and $R_3$ have the above meaning, are reacted with an alkali metal salt or with a quaternary ammonium salt of an acid of formula III

$$H\text{–}X' \qquad III$$

in which X' has the above meaning, in a solvent which is inert under the reaction conditions.

17. Method according to claim 16, characterized in that polyacyloxycyclopenta(c)pyran compounds according to claim 16 are produced, in which A, B and X' have the meaning given in claim 16, $R_2$ is isovaleroyl or β-methylvaleroyl, and of the radicals $R_1$ and $R_3$ one stands for acetyl and the other stands for isovaleroyl or β-acetoxyisovaleroyl or, if B = OH, also one stands for α-isovaleroyloxyisovaleroyl and the other stands for acetyl.

18. Method according to claim 16, characterized in that polyacyloxycyclopenta(c)pyran compounds according to claim 17 are produced, in which X' is a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

19. Method according to claim 16, characterized in that polyacyloxycyclopenta(c)pyran compounds according to claim 16 are produced, in which A and B are each are hydrogen and $R_1$, $R_2$, $R_3$ and X' have the meaning given in claim 16.

20. Method according to claim 16, characterized in that polyacyloxycyclopenta(c)pyran compounds according to claim 19 are produced, in which $R_1$, $R_2$, $R_3$, A and B have the meaning given in claim 19 and X' is halogen, alkanoyloxy with 2

to 10 carbon atoms or a benzoyloxy, phenylalkanoyloxy or phenylalkenoyloxy group with up to 3 carbon atoms in the alkanoyloxy or alkenoyloxy radical, which may be optionally substituted in the phenyl ring with halogen, lower alkoxy or lower alkyl.

21. Method according to claim 20, characterized in that polyacyloxycyclopenta(c)pyran compounds according to claim 20 are produced, in which $R_1$ is isovaleroyl, $R_2$ denotes isovaleroyl or β-methylvaleroyl and $R_3$ is acetyl and A, B and X have the meaning given in claim 20.

22. Method according to claim 21, characterized in that as compounds of formula Ia didrovaltrato- and/or homodidrovaltratohydrins according to claim 21 are produced, in which X' is chlorine or alkanoyloxy with 2 to 7 carbon atoms.

23. Method according to claim 21, characterized in that as compounds of formula Ia didrovaltrato- and/or homodidrovaltratohydrins according to claim 21 are produced, in which X' is a benzoyloxy or phenylacetoxy group, the phenyl ring of which may be optionally substituted with chlorine.

**Revendications pour les états contractants: BE, CH, DE, FR, GB, IT, LI, NL**

1. Utilisation de composés de polyacyloxycyclopenta(c)pyranne de formule générale I

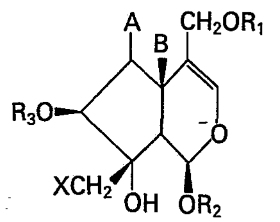

I

dans laquelle A est un hydrogène et B est un hydrogène ou un radical hydroxy, ou bien A et B forment ensemble une liaison, des radicaux $R_1$ à $R_3$, l'un est le radical isovaléroyle, un autre est le radical acétyle et le troisième est le radical acyle de l'un des acides suivants: acide isovalérique, acide β-méthylvalérique, acide α-isovaléroyloxyisovalérique, acide α-acétoxyisovalérique, acide β-acétoxyisovalérique, acide β-acétoxy-β-méthylvalérique ou acide β-hydroxyisovalérique, et X désigne un halogène ou un radical cyano, thiocyano, azido, ou un radical acyloxy $R_4COO$, où $R_4$ est un groupe alkyle, éventuellement substitué par un radical hydroxy, ayant de 1 à 20 atomes de carbone, ou un groupe alcényle ayant de 3 à 20 atomes de carbone, ou un groupe phényle, phénylalkyle ou phénylalcényle ayant jusqu'à 3 atomes de carbone dans la chaîne alkylène ou alcénylène, qui peut éventuellement être substitué sur le noyau phényle par halogène, trifluorométhyle, hydroxy, alkyle inférieur ou alcoxy inférieur, pour la préparation de compositions pharmaceutiques pour le traitement et la prophylaxie des ulcères peptiques et/ou pour le traitement de troubles de la motilité du type crampe dans les voies gastrointestinales chez les mammifères supérieurs et chez l'homme.

2. Utilisation de composés de polyacyloxycyclopenta(c)pyranne selon la revendication 1, caractérisée en ce qu'on utilise des composés de polyacyloxycyclopenta(c)pyranne de formule I dans laquelle $R_1$, $R_2$, $R_3$, A et B ont les significations données dans la revendication 1, et X est un halogène, un radical alcanoyloxy ayant de 2 à 10 atomes de carbone ou un radical benzoyloxy, phénylalcanoyloxy ou phénylalcénoyloxy ayant jusqu'à 3 atomes de carbone dans le fragment alcanoyloxy ou alcénoyloxy, qui peut éventuellement être substitué sur le noyau phényle par halogène, alcoxy inférieur ou alkyle inférieur.

3. Utilisation de composés de polyacyloxycyclopenta(c)pyranne selon la revendication 1 ou 2, caractérisée en ce qu'on utilise des composés de polyacyloxycyclopenta(c)pyranne de formule I dans laquelle A, B, $R_1$, $R_3$ et X ont les significations données dans la revendication 1 ou 2, et $R_2$ est le radical isovaléroyle ou β-méthylvaléroyle.

4. Utilisation de composés de polyacyloxycyclopenta(c)pyranne selon la revendication 3, caractérisée en ce qu'on utilise des composés de polyacyloxycyclopenta(c)pyranne dans lesquels A, B, X et $R_2$ ont les significations données dans la revendication 3 et, des radicaux $R_1$ et $R_2$, l'un est le radical acétyle et l'autre est le radical isovaléroyle ou β-acétoxyisovaléroyle ou bien encore, si B = OH, l'un est le radical α-isovaléroyloxyisovaléroyle et l'autre est le radical acétyle.

5. Utilisation de composés de polyacyloxycyclopenta(c)pyranne selon la revendication 1 ou 2, caractérisée en ce qu'on utilise des composés de polyacyloxycyclopenta(c)pyranne de formule I, dans laquelle A et B représentent chacun un hydrogène, et $R_1$, $R_2$, $R_3$ et X ont les significations données dans la revendication 1 ou 2.

6. Utilisation de composés de polyacyloxycyclopenta(c)pyranne selon la revendication 1, caractérisée en ce qu'on utilise des composés de polyacyloxycyclopenta(c)pyranne de formule I, dans laquelle A et B représentent chacun un hydrogène, $R_1$ est le radical isovaléroyle, $R_2$ le radical isovaléroyle ou β-méthylvaléroyle, et $R_3$ le radical acétyle, et X a la signification donnée dans la revendication 1.

7. Utilisation de composés de polyacyloxycyclopenta(c)pyranne selon la revendication 6, caractérisée en ce qu'on utilise des hydrines de didrovaltrate et/ou d'homodidrovaltrate, où X est un halogène, un radical alcanoyloxy ayant de 2 à 10 atomes de carbone ou un groupe benzoyloxy, phénylalcanoyloxy ou phénylalcénoyloxy ayant jusqu'à 3 atomes de carbone dans le fragment alcanoyloxy ou alcénoyloxy, qui peut éventuellement être substitué sur le noyau phényle par halogène, radical alcoxy inférieur ou alkyle inférieur.

8. Utilisation de composés de polyacyloxycyclopenta(c)pyranne selon la revendication 7, caractérisée en ce qu'on utilise de la didrovaltrate-chlorhydrine et/ou de l'homodidrovaltrate-chlorhydrine.

9. Utilisation de composés de polyacyloxycyclopenta(c)pyranne selon la revendication 7, caractérisée en ce qu'on utilise des didrovaltrate-alcanoy-

loxyhydrines et/ou des homodidrovaltrate-alcanoyloxyhydrines ayant un fragment alcanoyloxy ayant de 2 à 7 atomes de carbone.

10. Utilisation de composés de polyacyloxycyclopenta(c)pyranne selon la revendication 9, dans laquelle le radical alcanoyloxy est le radical isovaléroyloxy.

11. Utilisation de composés de polyacyloxycyclopenta(c)pyranne selon la revendication 7, caractérisée en ce qu'on utilise des hydrines de didrovaltrate et/ou d'homodidrovaltrate, où X est un groupe benzoyloxy ou phénylacétoxy, dont le noyau phényle peut être éventuellement substitué par le chlore.

12. Utilisation de composés de polyacyloxycyclopenta(c)pyranne selon la revendication 1, caractérisée en ce qu'on utilise des composés de formule I dans laquelle A est un hydrogène et B un radical hydroxy, X est un halogène, $R_2$ est un radical isovaléroyle ou β-méthylvaléroyle et, des radicaux $R_1$ et $R_3$, l'un est le radical acétyle et l'autre le radical isovaléroyle, β-acétoxyisovaléroyle ou α-isovaléroyloxyisovaléroyle.

13. Utilisation de composés de polyacyloxycyclopenta(c)pyranne selon la revendication 1 ou 2, caractérisée en ce qu'on utilise des composés de formule I dans laquelle A et B forment ensemble une liaison, et $R_1$, $R_2$, $R_3$ et X ont les significations données dans la revendication 1 ou 2.

14. Utilisation de composés de polyacyloxycyclopenta(c)pyranne selon la revendication 13, caractérisée en ce qu'on utilise des composés de formule I dans laquelle A et B forment ensemble une liaison, des radicaux $R_1$ et $R_3$, l'un est le radical acétyle et l'autre le radical isovaléroyle ou β-acétoxyisovaléroyle, et $R_2$ est le radical isovaléroyle ou β-methylvaléroyle, et X a la signification donnée dans la revendication 13.

15. Procédé pour la préparation de médicaments pour le traitement et la prophylaxie des ulcères peptiques et/ou pour le traitement de troubles de la motilité, du type crampe, dans les voies gastrointestinales, caractérisé en ce qu'on convertit en une forme médicamenteuse appropriée, des adjuvants pharmaceutiques habituels ensemble avec une quantité ayant un effet inhibiteur d'ulcère et/ou normalisateur de motilité, de composés de polyacyloxycyclopenta(c)pyranne selon la revendication 1, ou de leurs mélanges.

16. Dérivés de polyacyloxycyclopenta(c)pyranne de formule générale la

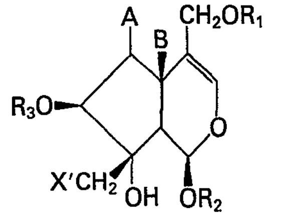

Ia

dans laquelle A est un hydrogène et B représente un hydrogène ou un radical hydroxy, ou bien A et B forment ensemble une liaison, des radicaux $R_1$ à $R_3$, l'un est le radical isovaléroyle, un autre est le radical acétyle et le troisième est le radical acyle de l'un des acides suivants: acide isovalérique, acide β-méthylvalérique, acide α-isovaléroyloxyisovalérique, acide α-acétoxyisovalérique, acide β-acétoxyisovalérique, acide β-acétoxy-β-méthylvalérique ou acide β-hydroxyisovalérique, et X' représente un radical cyano, azido ou un radical acyloxy $R_4COO$ dans lequel $R_4$ est un radical éthyle, propyle, isopropyle, n-butyle, 1-méthylpropyle ou tert-butyle éventuellement substitué par un radical hydroxy, un groupe alkyle, éventuellement substitué par un radical hydroxy, ayant de 5 à 20 atomes ce carbone, ou un groupe alcényle ayant de 3 à 20 atomes de carbone, ou un groupe phényle, phénylalkyle ou phénylalcényle ayant jusqu'à 3 atomes de carbone dans la chaîne alkylène ou alcénylène, qui peut éventuellement être substitué sur le noyau phényle par halogène, trifluorométhyle, hydroxy, alkyle inférieur ou alcoxy inférieur, ou bien, si B représente un radical hydroxy, et/ou $R_2$ n'est pas le radical isovaléroyle, et/ou quand l'un des radicaux $R_1$ et $R_3$ est un radical acétyle et l'autre n'est pas le radical isovaléroyle, X' peut aussi être un halogène ou un radical acétoxy ou thiocyano, ou bien A et B forment ensemble une liaison, X' est le chlore, et $R_1$ et $R_2$ représentent chacun un radical isovaléroyle et $R_3$ le radical acétyle, ou bien $R_1$ est le radical acétyle, $R_2$ le radical isovaléroyle et $R_3$ le radical acétoxyisovaléroyle.

17. Composés de polyacyloxycyclopenta(c)pyranne selon la revendication 16, dans lesquels A, B et X' ont les significations données dans la revendication 16, $R_2$ est le radical isovaléroyle ou β-méthylvaléroyle et, des radicaux $R_1$ et $R_3$, l'un est le radical acétyle et l'autre le radical isovaléroyle ou β-acétoxyisovaléroyle, ou bien, si B = OH, l'un est le radical α-isovaléroyloxyisovaléroyle et l'autre le radical acétyle.

18. Composés de polyacyloxycyclopenta(c)pyranne selon la revendication 17, dans lesquels X' représente un groupe benzoyloxy, phénylalcanoyloxy ou phénylalcénoyloxy ayant jusqu'à 3 atomes de carbone dans le fragment alcanoyloxy ou alcénoyloxy, qui peut être éventuellement substitué sur le noyau phényle par halogène, alcoxy inférieur ou alkyle inférieur.

19. Composés de polyacyloxycyclopenta(c)pyranne selon la revendication 16, dans lesquels A et B représentent chacun un hydrogène, et $R_1$, $R_2$, $R_3$ et X' ont les significations données dans la revendication 16.

20. Composés de polyacyloxycyclopenta(c)pyranne selon la revendication 19, dans lesquels $R_1$, $R_2$, $R_3$, A et B ont les significations données dans la revendication 19, et X' est un halogène ou un radical alcanoyloxy ayant de 2 à 10 atomes de carbone, ou un groupe benzoyloxy, phénylalcanoyloxy ou phénylalcénoyloxy ayant jusqu'à 3 atomes de carbone dans le fragment alcanoyloxy ou alcénoyloxy, qui peut être éventuellement substitué sur le noyau phényle par halogène, alcoxy inférieur ou alkyle inférieur.

21. Composés de polyacyloxycyclopenta(c)pyranne selon la revendication 20, dans lesquels $R_1$ est le radical isovaléroyle, $R_2$ le radical isovalé-

royle ou β-méthylvaléroyle, et $R_3$ est le radical acétyle, et A, B et X ont les significations données dans la revendication 20.

22. Didrovaltrate- et/ou homodidrovaltrate-hydrines selon la revendication 21, dans lesquelles X' est le chlore ou un radical alcanoyloxy ayant de 2 à 7 atomes de carbone.

23. Didrovaltrate- et/ou homodidrovaltrate-hydrines selon la revendication 21, dans lesquelles X' est un groupe benzoyloxy ou phénylacétoxy, dont le noyau phényle peut être éventuellement substitué par le chlore.

24. Procédé pour la préparation de composés de formule Ia

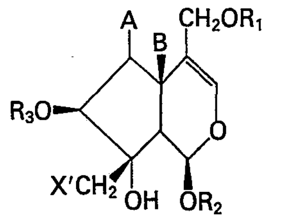

Ia

dans laquelle les substitants A, B, $R_1$–$R_3$ et X' ont les significations données dans la revendication 16, caractérisé en ce qu'on fait réagir des composés de formule II

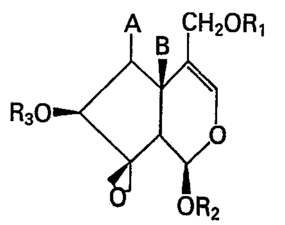

II

dans laquelle A, B, $R_1$, $R_2$ et $R_3$ ont les significations données ci-dessus, sur un sel de métal alcalin ou un sel d'ammonium quaternaire d'un acide de formule III

H–X' III

dans laquelle X' a la signification ci-dessus, dans un solvant inerte dans les conditions de la réaction.

25. Médicaments, caractérisés en ce qu'ils contiennent, outre les adjuvants pharmaceutiques habituels, à titre de principes actifs une quantité efficace de composés de polyacyloxycyclopenta(c)pyranne selon la revendication 16 ou de leurs mélanges.

26. Composés de polyacyloxycyclopenta(c)pyranne de formule générale Ib

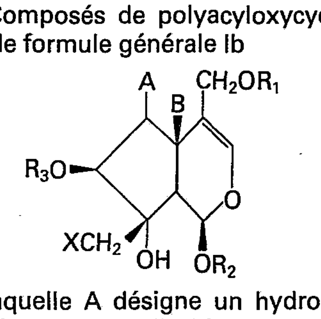

Ib

dans laquelle A désigne un hydrogène et B un hydrogène ou un radical hydroxy, ou bien A et B forment ensemble une liaison, des radicaux $R_1$ à $R_3$, l'un est le radical isovaléroyle, un autre le radical acétyle et le troisième le radical acyle de l'un des acides suivants: acide isovalérique, acide β-méthylvalérique, acide β-isovaléroyloxyisovalérique, acide α-acétoxyisovalérique, acide β-acétoxyisovalérique, acide α-acétoxy-β-méthylvalérique ou β-hydroxyisovalérique, et X est un halogène, ou un radical cyano, thiocyano, azido ou un radical acyloxy $R_4COO$ dans lequel $R_4$ est un groupe alkyle ayant de 1 à 20 atomes de carbone et qui, si A et B forment ensemble une liaison, contient au moins 6 atomes de carbone, un groupe alkyle éventuellement substitué par un groupe hydroxy et ayant de 1 à 20 atomes de carbone, un groupe alcényle ayant de 3 à 20 atomes de carbone ou un groupe phényle, phénylalkyle ou phénylalcényle ayant jusqu'à 3 atomes de carbone dans la chaîne alkylène ou alcénylène, et qui peut être éventuellement substitué sur le noyau phényle par halogène, trifluorométhyle, hydroxy, alkyle inférieur ou alcoxy inférieur, pour utilisation lors du traitement et de la prophylaxie des ulcères peptiques et/ou du traitement des troubles de la motilité, du type crampe, dans les voies gastrointestinales des mammifères supérieurs et de l'homme.

27. Composés de polyacyloxycyclopenta(c)pyranne selon la revendication 26, dans lesquels $R_1$, $R_2$, $R_3$, A et B ont les significations données dans la revendication 26, et X est un halogène, un groupe alcanoyloxy ayant de 2 à 10 atomes de carbone et qui, si A et B forment ensemble une liaison, contient au moins 7 atomes de carbone, ou bien un groupe benzoyloxy, phénylalcanoyloxy ou phénylalcénoyloxy ayant jusqu'à 3 atomes de carbone dans le fragment alcanoyloxy ou alcénoyloxy, qui peut être éventuellement substitué sur le noyau phényle par halogène, alcoxy inférieur ou alkyle inférieur.

28. Composés de polyacyloxycyclopenta(c)pyranne selon la revendication 26 ou 27, dans lesquels A, B, $R_1$, $R_3$ et X ont les significations données dans les revendications 26 ou 27, et $R_2$ est le radical isovaléroyle ou β-méthylvaléroyle.

29. Composés de polyacyloxycyclopenta(c)pyranne selon la revendication 28, dans lesquels A, B, X et $R_2$ ont les significations données dans la revendication 28 et, des radicaux $R_1$ et $R_3$, l'un est le radical acétyle et l'autre le radical isovaléroyle ou β-acétoxyisovaléroyle, ou bien, si B = OH, l'un est le radical α-isovaléroyloxyisovaléroyle et l'autre le radical acétyle.

30. Composés de polyacyloxycyclopenta(c)pyranne selon la revendication 26 ou 27, dans lesquels A et B représentent chacun un hydrogène, et $R_1$, $R_2$, $R_3$ et X ont les significations données dans les revendications 26 ou 27.

31. Composés de polyacyloxycyclopenta(c)pyranne selon la revendication 26, dans lesquels A et B représentent chacun un hydrogène, $R_1$ est le radical isovaléroyle, $R_2$ le radical isovaléroyle ou β-méthylvaléroyle et $R_3$ le radical acétyle, et X a la signification donnée dans la revendication 26.

32. Hydrines du didrovaltrate et/ou de l'homodidrovaltrate selon la revendication 31, dans lesquelles X est un halogène, un radical alcanoyloxy ayant de 2 à 10 atomes de carbone ou un groupe

benzoyloxy, phénylalcanoyloxy ou phénylalcénoyloxy ayant jusqu'à 3 atomes de carbone dans le fragment alcanoyloxy ou alcénoyloxy, qui peut éventuellement être substitué sur le noyau phényle par halogène, alcoxy inférieur ou alkyle inférieur.

33. Hydrines du didrovaltrate et/ou de l'homodidrovaltrate selon la revendication 32, dans lesquelles X est un groupe benzoyloxy ou phénylacétoxy, dont le noyau phényle peut être éventuellement substitué par le chlore.

34. Hydrines du didrovaltrate et/ou de l'homodidrovaltrate selon la revendication 32, dans lesquelles X est le chlore.

35. Hydrines du didrovaltrate et/ou de l'homodidrovaltrate selon la revendication 32, dans lesquelles X est un radical alcanoyloxy ayant de 2 à 7 atomes de carbone.

36. Hydrines du didrovaltrate et/ou de l'homodidrovaltrate selon la revendication 32, dans lesquelles X est le radical isovaléroyloxy.

37. Composés de polyacyloxycyclopenta(c)pyranne selon la revendication 26, dans lesquels A est un hydrogène et B le radical hydroxy, X est un halogène, $R_2$ représente le radical isovaléroyle ou β-méthylvaléroyle, et, des radicaux $R_1$ et $R_3$, l'un est le radical acétyle et l'autre le radical isovaléroyle, β-acétoxyisovaléroyle ou α-isovaléroyloxyisovaléroyle.

38. Composés de polyacyloxycyclopenta(c)pyranne selon la revendication 26 ou 27, dans lesquels A et B forment ensemble une liaison, et $R_1$, $R_2$, $R_3$ et X ont les significations données dans les revendications 26 ou 27.

39. Composés de polyacyloxycyclopenta(c)pyranne selon la revendication 38, dans lesquels A et B forment ensemble une liaison, des radicaux $R_1$ et $R_3$, l'un est le radical acétyle et l'autre le radical isovaléroyle ou β-acétoxyisovaléroyle, et $R_2$ est le radical isovaléroyle ou β-méthylvaléroyle, et X a la signification donnée dans la revendication 38.

**Revendications pour l'état contractant: AT**

1. Utilisation de composés de polyacyloxycyclopenta(c)pyranne de formule générale I

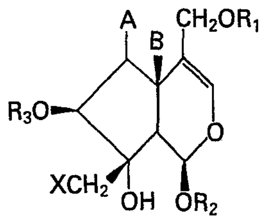

I

dans laquelle A désigne un hydrogène et B un hydrogène ou un radical hydroxy, ou bien A et B forment ensemble une liaison, des radicaux $R_1$ à $R_3$, l'un est le radical isovaléroyle, un autre le radical acétyle et le troisième le fragment acyle de l'un des acides suivants: acide isovalérique, acide β-méthylvalérique, acide α-isovaléroyloxyisovalérique, acide α-acétoxyisovalérique, acide β-acétoxyisovalérique, acide β-acétoxy-β-méthylvalérique ou acide β-hydroxyisovalérique, et X représente un halogène ou un radical cyano, thiocyano, azido, ou un radical acyloxy $R_4COO$, dans lequel $R_4$ est un groupe alkyle, éventuellement substitué par un radical hydroxy, ayant de 1 à 20 atomes de carbone, ou un groupe alcényle ayant de 3 à 20 atomes de carbone, ou un groupe phényle, phénylalkyle ou phénylalcényle ayant jusqu'à 3 atomes de carbone dans la chaîne alkylène ou alcénylène, qui peut être éventuellement substitué sur le noyau phényle par halogène, trifluorométhyle, hydroxy, alkyle inférieur ou alcoxy inférieur, pour la préparation de compositions pharmaceutiques pour le traitement et la prophylaxie des ulcères peptiques et/ou pour le traitement de troubles de la motilité du type crampe dans les voies gastrointestinales chez les mammifères supérieurs et chez l'homme.

2. Utilisation de composés de polyacyloxycyclopenta(c)pyranne selon la revendication 1, caractérisée en ce qu'on utilise des composés de polyacyloxycyclopenta(c)pyranne de formule I dans laquelle $R_1$, $R_2$, $R_3$, A et B ont les significations données dans la revendication 1, et X est un halogène, un radical alcanoyloxy ayant de 2 à 10 atomes de carbone ou un groupe benzoyloxy, phénylalcanoyloxy ou phénylalcénoyloxy ayant jusqu'à 3 atomes de carbone dans le fragment alcanoyloxy ou alcénoyloxy, qui peut éventuellement être substitué sur le noyau phényle par halogène, alcoxy inférieur ou alkyle inférieur.

3. Utilisation de composés de polyacyloxycyclopenta(c)pyranne selon les revendications 1 ou 2, caractérisée en ce qu'on utilise des composés de polyacyloxycyclopenta(c)pyranne de formule I dans laquelle A, B, $R_1$, $R_3$ et X ont les significations données dans les revendications 1 ou 2, et $R_2$ est le radical isovaléroyle ou β-méthylvaléroyle.

4. Utilisation de composés de polyacyloxycyclopenta(c)pyranne selon la revendication 3, caractérisée en ce qu'on utilise des composés de polyacyloxycyclopenta(c)pyranne dans lesquels A, B, X et $R_2$ ont les significations données dans la revendication 3 et, des radicaux $R_1$ et $R_3$, l'un est le radical acétyle et l'autre est le radical isovaléroyle ou β-acétoxyisovaléroyle ou bien, si B = OH, l'un représente le radical α-isovaléroyloxyisovaléroyle et l'autre est le radical acétyle.

5. Utilisation de composés de polyacyloxycyclopenta(c)pyranne selon la revendication 1 ou 2, caractérisée en ce qu'on utilise des composés de polyacyloxycyclopenta(c)pyranne de formule I, dans laquelle A et B représente chacun un hydrogène, et $R_1$, $R_2$, $R_3$ et X ont les significations données dans les revendications 1 ou 2.

6. Utilisation de composés de polyacyloxycyclopenta(c)pyranne selon la revendication 1, caractérisée en ce qu'on utilise des composés de polyacyloxycyclopenta(c)pyranne de formule I, dans laquelle A et B représentent chacun un hydrogène, $R_1$ est le radical isovaléroyle, $R_2$ le radical isovaléroyle ou β-méthylvaléroyle, et $R_3$ le radical acétyle, et X a la signification donnée dans la revendication 1.

7. Utilisation de composés de polyacyloxycyclopenta(c)pyranne selon la revendication 6, caractérisée en ce qu'on utilise des hydrines du didrovaltrate et/ou de l'homodidrovaltrate, dans lesquels X est un halogène ou un radical alcanoyloxy ayant de 2 à 10 atomes de carbone ou un groupe benzoyloxy, phénylalcanoyloxy ou phénylalcénoyloxy ayant jusqu'à 3 atomes de carbone dans le fragment alcanoyloxy ou alcénoyloxy, qui peut éventuellement être substitué sur le noyau phényle par halogène, alcoxy inférieur ou alkyle inférieur.

8. Utilisation de composés de polyacyloxycyclopenta(c)pyranne selon la revendication 7, caractérisée en ce qu'on utilise de la didrovaltrate-chlorhydrine et/ou de l'homodidrovaltrate-chlorhydrine.

9. Utilisation de composés de polyacyloxycyclopenta(c)pyranne selon la revendication 7, caractérisée en ce qu'on utilise des dihydrovaltrate-alcanoyloxyhydrines et/ou des homodidrovaltrate-alcanoyloxyhydrines ayant un radical alcanoyle ayant de 2 à 7 atomes de carbone.

10. Utilisation de composés de polyacyloxycyclopenta(c)pyranne selon la revendication 9, dans laquelle le radical alcanoyloxy représente le radical isovaléroyloxy.

11. Utilisation de composés de polyacyloxycyclopenta(c)pyranne selon la revendication 7, caractérisée en ce qu'on utilise des hydrines du didrovaltrate et/ou de l'homodidrovaltrate, où X est un groupe benzoyloxy ou phénylacétoxy, dont le noyau phényle peut être éventuellement substitué par le chlore.

12. Utilisation de composés de polyacyloxycyclopenta(c)pyranne selon la revendication 1, caractérisée en ce qu'on utilise des composés de formule I dans laquelle A est un hydrogène et B représente un radical hydroxy, X est un halogène, $R_2$ est un radical isovaléroyle ou β-méthylvaléroyle et, des radicaux $R_1$ et $R_3$, l'un est le radical acétyle et l'autre le radical isovaléroyle, β-acétoxyisovaléroyle ou α-isovaléroyloxyisovaléroyle.

13. Utilisation de composés de polyacyloxycyclopenta(c)pyranne selon la revendication 1 ou 2, caractérisée en ce qu'on utilise des composés de formule I dans laquelle A et B forment ensemble une liaison, et $R_1$, $R_2$, $R_3$ et X ont les significations données dans les revendications 1 ou 2.

14. Utilisation de composés de polyacyloxycyclopenta(c)pyranne selon la revendication 13, caractérisée en ce qu'on utilise des composés de formule I dans laquelle A et B forment ensemble une liaison, des radicaux $R_1$ et $R_3$, l'un est le radical acétyle et l'autre le radical isovaléroyle ou β-acétoxyisovaléroyle, et $R_2$ représente le radical isovaléroyle ou β-methylvaléroyle, et X a la signification donnée dans la revendication 13.

15. Procédé pour la préparation de médicaments pour le traitement et la prophylaxie des ulcères peptiques et/ou pour le traitement de troubles de la motilité, du type crampe, dans les voies gastrointestinales, caractérisé en ce qu'on convertit en une forme médicamenteuse appropriée, ayent un effet adjuvants pharmaceutiques habituels, une quantité, à effet inhibiteur d'ulcère et/ou normalisateur de motilité, de composés de polyacyloxycyclopenta(c)pyranne selon la revendication 1, ou de leurs mélanges.

16. Procédé pour la préparation de composés de polyacyloxycyclopenta(c)pyranne de formule générale Ia

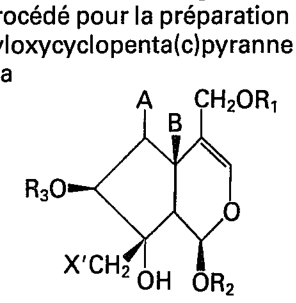

Ia

dans laquelle A est un hydrogène et B représente un hydrogène ou le radical hydroxy, ou bien A et B forment ensemble une liaison, des radicaux $R_1$ à $R_3$, l'un est le radical isovaléroyle, un autre est le radical acétyle et le troisième est le fragment acyle de l'un des acides suivants: acide isovalérique, acide β-méthylvalérique, acide α-isovaléroyloxyisovalérique, acide α-acétoxyisovalérique, acide β-acétoxyisovalérique, acide β-acétoxy-β-méthylvalérique ou acide β-hydroxyisovalérique, et X' représente un radical cyano, azido ou un radical acyloxy $R_4COO$ dans lequel $R_4$ représente un radical éthyle, propyle, isopropyle, n-butyle, 1-méthylpropyle ou tert-butyle éventuellement substitué par un groupe hydroxy, un groupe alkyle, éventuellement substitué par un radical hydroxy, ayant de 5 à 20 atomes ce carbone, ou un groupe alcényle ayant de 3 à 20 atomes de carbone, ou un groupe phényle, phénylalkyle ou phénylalcényle ayant jusqu'à 3 atomes de carbone dans la chaîne alkylène ou alcénylène, qui peut être éventuellement substitué sur le noyau phényle par halogène, trifluorométhyle, hydroxy, alkyle inférieur ou alcoxy inférieur, ou bien, si B représente un radical hydroxy, et/ou si $R_2$ n'est pas le radical isovaléroyle, et/ou si l'un des radicaux $R_1$ et $R_3$ est le radical acétyle et l'autre n'est pas le radical isovaléroyle, X' peut aussi être un halogène ou un radical acétoxy ou thiocyano, ou bien A et B forment ensemble une liaison, X' est le chlore, et $R_1$ et $R_2$ représentent chacun un radical isovaléroyle et $R_3$ le radical acétyle, ou bien $R_1$ est le radical acétyle, $R_2$ le radical isovaléroyle et $R_3$ le radical acétoxyisovaléroyle, caractérisé en ce qu'on fait réagir des composés de formule II

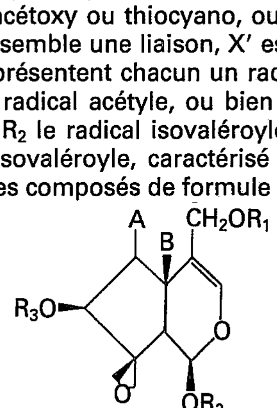

II

dans laquelle A, B, $R_1$, $R_2$ et $R_3$ ont la signification ci-dessus, sur un sel de métal alcalin ou un sel d'ammonium quaternaire d'un acide de formule III

$$H\text{–}X' \qquad III$$

dans laquelle X' a la signification donnée ci-dessus, dans un solvant inerte dans les conditions de la réaction.

17. Procédé selon la revendication 16, caractérisé en ce qu'on prépare des composés de polyacyloxycyclopenta(c)pyranne selon la revendication 16, dans lesquels A, B et X' ont les significations données dans la revendication 16, $R_2$ est le radical isovaléroyle ou β-méthylvaléroyle, et, des radicaux $R_1$ et $R_3$, l'un est le radical acétyle et l'autre le radical isovaléroyle ou β-acétoxyisovaléroyle, ou bien, si B = OH, l'un est le radical α-isovaléroyloxyisovaléroyle et l'autre le radical acétyle.

18. Procédé selon la revendication 16, caractérisé en ce qu'on prépare des composés de polyacyloxycyclopenta(c)pyranne selon la revendication 17, dans lesquels X' est un groupe benzoyloxy, phénylalcanoyloxy ou phénylalcénoyloxy ayant jusqu'à 3 atomes de carbone dans le fragment alcanoyloxy ou alcénoyloxy, qui peut éventuellement être substitué sur le noyau phényle par halogène, acyloxy inférieur ou alkyle inférieur.

19. Procédé selon la revendication 16, caractérisée en ce qu'on prépare des composés de polyacyloxycyclopenta(c)pyranne selon la revendication 16, dans lesquels A et B sont chacun un hydrogène, et $R_1$, $R_2$, $R_3$ et X' ont les significations données dans la revendication 16.

20. Procédé selon la revendication 16, caractérisée en ce qu'on prépare des composés de polyacyloxycyclopenta(c)pyranne selon la revendication 19, dans lesquels $R_1$, $R_2$, $R_3$, A et B ont les significations données dans la revendication 19, et X' est un halogène, ou un radical alcanoyloxy ayant de 2 à 10 atomes de carbone, ou un groupe benzoyloxy, phénylalcanoyloxy ou phénylalcénoyloxy ayant jusqu'à 3 atomes de carbone dans le fragment alcanoyloxy ou alcénoyloxy, qui peut être éventuellement substitué sur le noyau phényle par halogène, alcoxy inférieur ou alkyle inférieur.

21. Procédé selon la revendication 1, caractérisée en ce qu'on prépare des composés de polyacyloxycyclopenta(c)pyranne selon la revendication 20, dans lesquels $R_1$ est le radical isovaléroyle, $R_2$ le radical isovaléroyle ou β-méthylvaléroyle et $R_3$ le radical acétyle, et A, B et X ont les significations données dans la revendication 20.

22. Procédé selon la revendication 21, caractérisée en ce qu'on prépare comme composés de formule Ia des didrovaltrate- et/ou homodidrovaltrate-hydrines selon la revendication 21, dans lesquelles X' est le chlore ou un radical alcanoyloxy, ayant de 2 à 7 atomes de carbone.

23. Procédé selon la revendication 21, caractérisée en ce qu'on prépare comme composé de formule Ia des didrovaltrate- et/ou des homodidrovaltrate-hydrines selon la revendication 21, dans lesquelles X' est un groupe benzoyloxy ou phénylacétoxy dont le noyau phényle peut être éventuellement substitué par le chlore.